# EUROPEAN PATENT SPECIFICATION

(11) **EP 3 423 441 B1**
(45) Date of publication and mention of the grant of the patent: **27.10.2021**
(21) Application number: 17713146.3
(22) Date of filing: 28.02.2017
(51) Int. Cl.: C07D 333/28, C07C 211/62, C07D 453/02, C07D 295/073, C07D 295/084, C07D 213/65, A61K 31/452, A61P 9/12, A61P 25/22, A61P 35/00, A61P 37/00

(54) **IODINATED ANALOGS OF CHOLINE, METHODS OF PREPARATION THEREOF, AND USE THEREOF AS MEDICAMENTS**
JODIERTE ANALOGA VON CHOLIN, VERFAHREN ZUR HERSTELLUNG DAVON UND VERWENDUNG DAVON ALS ARZNEIMITTEL
ANALOGUES IODÉS DE CHOLINE, PROCÉDÉS DE PRÉPARATION CORRESPONDANTS ET UTILISATION CORRESPONDANTE COMME MÉDICAMENTS

(30) Priority: 29.02.2016 CZ 20160117
(43) Date of publication of application: 09.01.2019
(73) Proprietor: Univerzita Palackého v Olomouci, 77147 Olomouc (CZ)
(72) Inventor: SVEC, Pavel, 37001 Ceske Budejovice (CZ); HRUBY, Martin, 16000 Praha 6 (CZ); KUCKA, Jan, 26265 Holubice-Kozinec (CZ); SEDLACEK, Ondrej, 16200 Praha 6 (CZ); PETRIK, Milos, 78301 Olomouc (CZ); NOVY, Zbynek, 77900 Olomouc (CZ); HAJDUCH, Marian, 79305 Moravsky Beroun (CZ)
(74) Representative: Hartvichova, Katerina
(86) International application number: PCT/CZ2017/050010
(87) International publication number: WO 2017/148455

(56) References cited:
- WO-A2-2012/040133
- GB-A- 881 265
- US-A1- 2007 043 234
- JAMES H. SHORT ET AL: "Sympathetic Nervous System Blocking Agents", JOURNAL OF PHARMACEUTICAL SCIENCES, vol. 51, no. 9, 1 September 1962 (1962-09-01), pages 881-884, XP055365888, WASHINGTON, US ISSN: 0022-3549, DOI: 10.1002/jps.2600510915
- ROBERT A. MOSS ET AL: "Iodosobenzoate-functionalized surfactant vesicles: adjustable reactivity in reactive phosphate cleavage", TETRAHEDRON LETTERS, vol. 30, no. 16, 1 January 1989 (1989-01-01), pages 2071-2074, XP055365755, AMSTERDAM, NL ISSN: 0040-4039, DOI: 10.1016/S0040-4039(01)93714-1

## Description

### Field of Art

The present invention relates to novel aromatic analogs of choline, to synthesis of these analogs, preparation of radiolabeled forms thereof and the medical use thereof as imaging agents and therapeutics, especially in oncological and inflammatory diseases.

### Background Art

The treatment of tumorous diseases greatly profits from the tools of nuclear medicine, including functional imaging methods; such as planar scintigraphy, positron emission tomography (PET), and single-photon emission computed tomography (SPECT) [Zaidi H. (Ed.) (2006), Quantitative Analysis in Nuclear Medicine Imaging, Springer US, 1st Issue, ISBN 978-0-387-25444-9; Welch M. J., Redvanly C. S. (2003), Handbook of Radiopharmaceuticals: Radiochemistry and Applications, John Wiley & Sons, Ltd. ISBN: 0-471-49560-3]. The above-mentioned functional imaging methods, in contrast to anatomical imaging methods (computer tomography - CT, magnetic resonance - MRI), allow visualization of biochemical changes in tissues, and are therefore used for diagnosis of various diseases [Pimlott S. L., Sutherland A. (2011), Chem. Soc. Rev. 40, 149-162].

Planar scintigraphy, positron emission tomography, and single-photon emission computed tomography all rely on radioactively labeled imaging agents. Unfortunately, many imaging agents suffer from drawbacks, such as a very short half-life of the used radioisotopes, complicated synthesis, or unsuitable distribution in the organism [Pimlott S. L., Sutherland A. (2011), Chem. Soc. Rev. 40, 149-162]. Only a few radiopharmaceuticals that are used for example in oncology allow a theranostic approach, which means that the same molecule can be used for both diagnostic and therapeutic purposes. Theranostic approach allows a significantly more accurate monitoring of dosage, of distribution of the radiopharmaceutical in the organism, and of the results of the treatment [Velikyan I. (2012), Theranostics 2, 424-426; Del Vecchio S. et al. (2007) J. Nucl. Med. Mol. Imaging 51, 152-63]. Examples of medicaments which enable the theranostic approach include several radioiodinated pharmaceuticals that are commonly labeled by ¹²³I or ¹³¹I for diagnostic purposes and by ¹³¹I for therapeutic purposes [Giammarile F. et al. (2008), Eur. J. Nucl. Med. Mol. Imaging 35, 1039-1047]. Choline transporters represent a molecular structure suitable for targeting diagnostic and therapeutic radiopharmaceuticals, as they are overexpressed on the surface of certain types of tumor cells, in particular prostate carcinoma, small-cell lung carcinoma, and glioma [Hara T. et al. (2006), Mol. Imaging 5, 498-509; Inazu M. et al. (2013), Pharmacol. Res. 76, 119-131]. This fact is already exploited in the clinical practice for visualization of certain types of tumor focuses, particularly prostate carcinoma. Three radiolabeled derivatives of choline - [¹⁸F]fluoromethylcholine, [¹⁸F]fluoroethylcholine, and [¹¹C]-choline - are currently employed in tumor imaging [Umbehr M. H. et al. (2013), Eur. Urol. 64, 106-117; Evangelista L. et al. (2015), Nucl. Med. Biol. 42, 340-348]. These compounds preferentially accumulate in tumor cells which overexpress choline transporters. [¹⁸F]Fluoromethylcholine, [¹⁸F]fluoroethylcholine, and [¹¹C]choline contain positron emitters in their molecules. The emitted positron provides - by annihilation with an electron - an antiparallel pair of photons. These antiparallel photons are captured by a detector, and the resulting data are then used for reconstruction of the three-dimensional image of the tumor focus [Pimlott S. L., Sutherland A. (2011), Chem. Soc. Rev. 40, 149-162].

Expression of choline transporters is also increased in a number of other tissues - in myocardium, digestive tract, neural tissue, in endocrine glands, in bone marrow and blood, in the skin, and in the lungs. Pathological function or expression of the choline transporters thus accompanies a number of human diseases - not only tumors, but for example also neuro-muscular diseases [Barwick K. E. S. et al. (2012), Am. J. Hum. Genet. 91, 1103-1107], atherosclerosis [Neumann S. A. et al. (2012), Int. J. Cardiovasc. Imaging 28, 243-250], alcohol addiction [Stankiewitz A. M. et al. (2015), Pharmacol. Biochem. Behav. 139 Part A, 27-38], and psychiatric disorders, such as depression [Hahn M. K. et al. (2008), Genes Brain Behav. 7, 487-495] or attention deficit disorder [English B. A. et al. (2009), J. Neurodev. Disord. 1, 252-263].

However, the above-mentioned choline derivatives used in clinical practice, suffer from serious drawbacks. Due to the very short half-life of ¹¹C *(t₁ₗ₂* = 20 min) and ¹⁸F (*t*_{*1*/*2*} = 110 min) an on-site cyclotron is necessary for a successful application of such medicaments to the patient. The short half-life also imposes strict requirements on the synthesis of the radiopharmaceutical and on the production quality control. Furthermore, in the visualization of prostate carcinoma, the short half-life of [¹⁸F]fluoromethylcholine, [¹⁸F]fluoroethylcholine, and [¹¹C]-choline causes problems in combination with rapid renal excretion [Bauman G. et al. 2012, Prostate Cancer Prostatic Dis. 15, 45-55]. The image quality is significantly decreased by background activity accumulated in the bladder and the short half-life does not allow acquiring the image after most of the radioactivity has been eliminated from the bladder. Due to the characteristics of the emitted radiation [¹⁸F]fluoromethylcholine, [¹⁸F]fluoroethylcholine, and [¹¹C]choline are applicable only for diagnostic purposes, and they cannot be used in therapy.

### Disclosure of the Invention

Object of the present invention are compounds of general formula I: 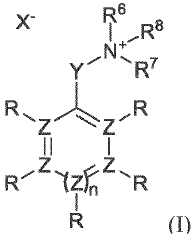 wherein
- the ring containing Z atoms is selected from benzene ring or thiophene ring;
- R are independently selected from the group consisting of H; ¹²³I; ¹²⁴I; ¹²⁵I; ¹²⁷I; ¹³¹I; C₁ to C₁₄ alkyl; C₂ to C₁₄ alkenyl; C₂ to C₁₄ alkynyl; C₆ to C₁₀ aryl; C₃ to C₆ heteroaryl comprising at least one heteroatom selected from O, S, N; C₃ to C₆ heterocyclyl comprising one to two heteroatoms selected from O, S, N; C₁ to C₁₄ alkoxy; C₆ to C₁₀ aryloxy; C₃ to C₆ heteroaryloxy comprising at least one heteroatom selected from O, S, N; C₃ to C₆ heterocyclyloxy comprising one to two heteroatoms selected from O, S, N; benzyloxy; C₁ to C₁₄ alkylthio; halogen; OH; SH; NH_{2;} C₁ to C₁₄ alkylamino; C₆ to C₁₀ arylamino; C₁ to C₁₄ acylamino; di(C₁ to C₁₄ alkyl)amino, wherein the alkyls are the same or different; di(C₆ to C₁₀ aryl)amino, wherein the aryls are the same or different; di(C₁ to C₁₄ acyl)amino, wherein the acyls are the same or different; CN; nitro and COORₙ, wherein Rₙ is H or C₁ to C₁₄ alkyl or aryl;
- whereas at least one of the substituents R is ¹²³I; ¹²⁴I; ¹²⁵I; or ¹³¹I,
- R⁶ is 2-hydroxyethyl, 3-hydroxypropyl, or 4-hydroxybutyl;
- R⁷ and R⁸ are independently selected from the group consisting of C₁ to C₆ alkyl; C₂ to C₆ alkenyl; C₂ to C₆ alkynyl; C₃ to C₈ cycloalkyl; C₃ to C₈ cycloalkenyl; C₆ to C₁₀ aryl; C₃ to C₆ heteroaryl comprising at least one heteroatom selected from O, S, N; C₃ to C₆ heterocyclyl comprising one or two heteroatoms selected from O, S, N; whereas R⁷ and R⁸ can optionally be substituted by OH group or C₁-C₄ hydroxyalkyl group; or
- R⁷ and R⁸ together with the nitrogen atom form a four- to seven-membered ring, optionally comprising at least one further heteroatom selected from O, S, N, a optionally substituted by OH group or C₁-C₄ hydroxyalkyl group; or
- whereas at least one, preferably exactly one, of substituents R⁶, R⁷ and R⁸ is substituted by OH group or by C₁-C₄ hydroxyalkyl group;
- Y is a linker formed by a linear or branched hydrocarbon chain having 1 to 10 carbon atoms, whereas optionally 1 to 2 carbon atoms can be replaced by oxygen and/or sulfur atoms; and
- X⁻ is a pharmaceutically acceptable anion;
with the proviso that:
- when exactly one of the substituents R is ¹²³I; ¹²⁴I; ¹²⁵I; ¹²⁷I or ¹³¹I, and is in the *ortho* position, and all other substituents R are hydrogen atoms, Y is -CH₂-, R⁶ is 2-hydroxyethyl, then NR⁷R⁸ is not dimethylamino or pyrrolidino group,
- when exactly one of the substituents R is ¹²³I; ¹²⁴I; ¹²⁵I; ¹²⁷I or ¹³¹I and is in the *ortho* position, and all other substituents R are hydrogen atoms, Y is -CH₂-, R⁶ is 2-hydroxyethyl, R⁷ is ethyl or 2-hydroxyethyl, then R⁸ is not methyl.

The general formula I of the present invention is meant to include all enantiomers and diastereomers.

Preferably, when R⁷ and R⁸ together with the nitrogen atom form a four- to seven-membered ring, this ring is substituted by OH or C₁-C₄ hydroxyalkyl group in the position 3 or 4.

Preferably, R⁷ and R⁸ together with the nitrogen atom for a five- to six-membered ring.

Preferably, when R⁶, R⁷ and R⁸ together with the nitrogen atom form a bicyclic group, this group is substituted by OH or C₁-C₄ hydroxyalkyl group in the position 3 or 4.

Preferably, the alkyl chains comprise 1 to 10 carbon atoms, or 1 to 8 carbon atoms, or 1 to 6 carbon atoms, or 1 to 4 carbon atoms.

Preferably, the alkenyl or alkynyl chains comprise 2 to 10 carbon atoms, or 2 to 8 carbon atoms, or 2 to 6 carbon atoms, or 2 to 4 carbon atoms.

Preferably, Y is a linear or branched hydrocarbon chain having 1 to 10 carbon atoms, whereas 1 carbon atom is replaced by oxygen; or wherein Y is linear hydrocarbon chain containing 1 to 3 carbon atoms.

Preferably, substituents R are independently selected from the group consisting of H; ¹²³I; ¹²⁴I; ¹²⁵I; ¹²⁷I; ¹³¹I; C₆ to C₁₀ aryl; C₁ to C₁₄ alkoxy; benzyloxy; NO₂.

Pharmaceutically acceptable salts (anions, cations) include inorganic or organic anions, inorganic or organic cations, addition salts with inorganic or organic acids or bases, co-crystals, inclusion compounds and other salts suitable for physiological administration [Stahl P. H., Wermuth, C. G. (Ed.) (2011), Pharmaceutical Salts: Properties, Selection, and Use, Wiley-VCH, 2. Vydání, ISBN 978-3-90639-051-2].

Suitable pharmaceutically acceptable anions according to the present invention preferably include anions derived from inorganic acids such as hydrochloric acid, hydrobromic acid, hydrofluoric acid, boric acid, tetrafluoroboric acid, phosphoric acid, nitric acid, carbonic acid, sulfuric acid, sulfurous acid; and from organic acids including alifatic, cycloalifatic, aromatic, arylalifatic, heterocyclic, carboxylic and sulfonic acids. Suitable organic anions are preferably selected from the group comprising acetate, isethionate, lactobionate, methanesulfonate, trifluoromethanesulfonate, 4-methylbenzenesulfonate, trifluoroacetate, formiate, propionate, succinate, glycolate, gluconate, digluconate, lactate, malate, tartrate, citrate, ascorbate, glucuronate, maleate, fumarate, pyruvate, aspartate, glutamate, benzoate, anthranilate, stearate, salicylate, 4-hydroxybenzoate, phenylacetate, mandelate, pamoate, ethanesulfonate, benzenesulfonate, panthotenate, 2-hydroxyethanesulfonate, sulfanilate, *N*-cyclohexylsulfamate, 3-hydroxybutyrate, galactarate, galacturonate, adipate, alginate, butyrate, camphorate, camphorsulfonate, cyclopentanepropionate, dodecylsulfate, glycoheptanoate, glycerolfosfate, heptanoate, hexanoate, nicotinate, 2-naphthalenesulfonate, oxalate, pectinate, 3-phenylpropionate, picrate, pivalate, thiocyanate, octanoate, decanoate, undecanoate, oxoglutarate, cinnamate, 1-hydroxy-2-naphthoate, 4-acetamidobenzoate, gentisate, glucoheptanoate, hippurate, isobutyrate, undecylenate, pyroglutamate, naphthalene-1,5-disulfonate, laurate, oleate, sebacate, pentanoate and palmitate.

In a preferred embodiment, the invention relates to the following derivatives of general formula I:
(2-hydroxyethyl)-[2-(3-iodo-4-methoxyphenyl)ethyl]-dimethylammonium
4-methylbenzenesulfonate (1),
(2-hydroxyethyl)-[2-(3,5-diiodo-4-methoxyphenyl)ethyl]-dimethylammonium 4-methylbenzenesulfonate (3),
(2-hydroxyethyl)-[3-(3-iodo-4-methoxyphenyl)propyl]-dimethylammonium 4-methylbenzenesulfonate (4),
(2-hydroxyethyl)-[2-(4-iodophenyl)ethyl] -dimethylammonium 4-methylbenzenesulfonate (5),
1-(2-hydroxyethyl)-1-[2-(4-iodophenyl)ethyl]pyrrolidin-1-ium 4-methylbenzenesulfonate (7),
(2-hydroxyethyl)-(3-iodobenzyl)-dimethylammonium methanesulfonate (9),
1-(2-hydroxyethyl)-1-(3-iodobenzyl)pyrrolidin-1-ium methanesulfonate (10),
(2-hydroxyethyl)-(4-iodobenzyl)-dimethylammonium bromide (11),
diethyl-(2-hydroxyethyl)-(4-iodobenzyl)ammonium bromide (12),
1-(2-hydroxyethyl)-1-(4-iodobenzyl)pyrrolidin-1-ium bromide (13),
1-(2-hydroxyethyl)-1-(2-iodobenzyl)piperidin-1-ium 4-methylbenzenesulfonate (14),
diethyl-(2-hydroxyethyl)-(2-iodobenzyl)ammonium 4-methylbenzenesulfonate (15),
1,4-bis(2-hydroxyethyl)-1-(2-iodobenzyl)piperazin-1-ium 4-methylbenzenesulfonate (17),
[3-(4-hexyloxy-3-iodophenyl)propyl]-(2-hydroxyethyl)-dimethylammonium 4-methylbenzensulfonate (21),
[3-(4-decyloxy-3-iodophenyl)propyl]-(2-hydroxyethyl)-dimethylammonium methanesulfonate (22),
(2-hydroxyethyl)-[2-(2-iodophenoxy)ethyl]-dimethylammonium bromide (23),
1-(2-hydroxyethyl)-1-[2-(2-iodophenoxy)ethyl]pyrrolidin-1-ium bromide (24),
(2-hydroxyethyl)-[2-(4-iodophenoxy)ethyl]-dimethylammonium bromide (25),
1-(2-hydroxyethyl)-1-[2-(4-iodophenoxy)ethyl]pyrrolidin-1-ium bromide (26),
(2-hydroxyethyl)-[2-(3-iodophenoxy)ethyl]-dimethylammonium bromide (27),
1-(2-hydroxyethyl)-1-[2-(3-iodophenoxy)ethyl]pyrrolidin-1-ium bromide (28),
(2-hydroxyethyl])-[6-(3-iodophenoxy)hexyl)-dimethylammonium bromide (29),
1-(2-hydroxyethyl)-1-[6-(3-iodophenoxy)hexyl]pyrrolidin-1-ium bromide (30),
(2-hydroxyethyl)-[4-(2-iodophenoxy)butyl]-dimethylammonium bromide (31),
1-(2-hydroxyethyl)-1-[4-(2-iodophenoxy)butyl]pyrrolidin-1-ium bromide (32),
(2-hydroxyethyl)-[2-(5-iodothiophen-2-yl)ethyl]-dimethylammonium 4-methylbenzenesulfonate (33),
1-(2-hydroxyethyl)-1-[2-(5-iodothiophen-2-yl)ethyl]pyrrolidin-1-ium 4-methylbenzenesulfonate (34),
4-(2-hydroxyethyl)-4-(4-iodobenzyl)morpholin-4-ium bromide (35),
4-(2-hydroxyethyl)-4-(2-iodobenzyl)morpholin-4-ium bromide (36),
(2-hydroxyetliyl)-[6-(4-iodo-3-nitrophenoxy)hexyl]-dimethylammonium bromide (40).

In another aspect, object of the present invention are compounds of general formula I for use as medicaments and/or diagnostic agents, preferably for use as theranostic agents.

Object of the present invention is also a medical preparation comprising at least one compound of general formula I according to the present invention, optionally in the form of a pharmaceutically acceptable salt or solvate, and at least one pharmaceutically acceptable carrier, filler and/or diluent.

A further object of the present invention are compounds of general formula Ia 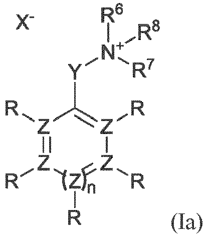 wherein
- the ring containing Z atoms is selected from benzene ring or thiophene ring;
- R are independently selected from the group consisting of H; ¹²⁷I; C₁ to C₁₄ alkyl; C₂ to C₁₄ alkenyl; C₂ to C₁₄ alkynyl; C₆ to C₁₀ aryl; C₃ to C₆ heteroaryl comprising at least one heteroatom selected from O, S, N; C₃ to C₆ heterocyclyl comprising one to two heteroatoms selected from O, S, N; C₁ to C₁₄ alkoxy; C₆ to C₁₀ aryloxy; C₃ to C₆ heteroaryloxy comprising at least one heteroatom selected from O, S, N; C₃ to C₆ heterocyclyloxy comprising one to two heteroatoms selected from O, S, N; benzyloxy; C₁ to C₁₄ alkylthio; halogen; OH; SH: NH₂; C₁ to C₁₄ alkylamino; C₆ to C₁₀ arylamino; C₁ to C₁₄ acylamino; di(C₁ to C₁₄ alkyl)amino, wherein the alkyls are the same or different; di(C₆ to C₁₀ aryl)amino, wherein the aryls are the same or different; di(C₁ to C₁₄ acyl)amino, wherein the acyls are the same or different; CN; nitro and COORₙ, wherein Rₙ is H or C₁ to C₁₄ alkyl or aryl;
- whereas at least one of the substituents R is ¹²⁷I,
- R⁶ is 2-hydroxyethyl, 3-hydroxypropyl, or 4-hydroxybutyl,
- R⁷ and R⁸ are independently selected from the group consisting of C₁ to C₆ alkyl; C₂ to C₆ alkenyl; C₂ to C₆ alkynyl; C₃ to C₈ cycloalkyl; C₃ to C₈ cycloalkenyl; C₆ to C₁₀ aryl; C₃ to C₆ heteroaryl comprising at least one heteroatom selected from O, S, N; C₃ to C₆ heterocyclyl comprising one or two heteroatoms selected from O, S, N; whereas R⁷ and R⁸ can optionally be substituted by OH group or C₁-C₄ hydroxyalkyl group; or
- R⁷ and R⁸ together with the nitrogen atom form a four- to seven-membered ring, optionally comprising at least one further heteroatom selected from O, S, N, and optionally substituted by OH group or C₁-C₄ hydroxyalkyl group; or
- whereas at least one, preferably exactly one, of substituents R⁶, R⁷ and R⁸ is substituted by OH group or by C₁-C₄ hydroxyalkyl group;
- Y is a linker formed by a linear or branched hydrocarbon chain having 1 to 10 carbon atoms, whereas optionally 1 carbon atom can be replaced by an oxygen or sulfur atom; and
- X⁻ is a pharmaceutically acceptable anion;
with the proviso that:
- when exactly one of the substituents R is ¹²⁷I, and is in the *ortho* position, and all other substituents R are hydrogen atoms, Y is -CH₂-, R⁶ is 2-hydroxyethyl, then NR⁷R⁸ is not dimethylamino or pyrrolidino group,
- when exactly one of the substituents R is ¹²⁷I and is in the *ortho* position, and all other substituents R are hydrogen atoms, Y is -CH₂-, R⁶ is 2-hydroxyethyl, R⁷ is ethyl or 2-hydroxyethyl, then R⁸ is not methyl.

The general fonnula Ia is meant to include all enantiomers and diastereomers. The preferred embodiments listed for formula I are applicable also for formula Ia.

Disorders involving pathological function or expression of choline transporters include in particular oncologic diseases, such as prostate carcinoma, lung carcinoma, glioma; inflammatory, neurological and neuromuscular disorders; and atherosclerosis.

Another object of the present invention is use of the compounds of general formula Ia according to the present invention for *in vitro* diagnostics.

The compounds of the present invention may be prepared by alkylation of a tertiary amine using a corresponding alkylation agent.

The present invention provides a novel group of aromatic choline analogs which may be radiolabeled by various iodine isotopes. The novel choline analogs overcome the drawbacks of [¹⁸F]fluoromethylcholine, [¹⁸F]fluoroethylcholine, and [¹¹C]choline as described above, because they use pharmaceutically relevant iodine radioisotopes with significantly longer half-lives. By an appropriate selection of the iodine isotope [Coenen H. H. et al. (2006), Radioiodination Reactions for Pharmaceuticals: Compendium for Effective Synthesis Strategies, Springer Netherlands, 1st Ed.], radiopharmaceuticals applicable not only for positron emission tomography (¹²⁴I) but also for single-photon computed emission tomography (¹²³I, ¹²⁵I, ¹³¹I), planar scintigraphy (¹²³I, ¹²⁵I, ¹³¹I), as well as for therapy (¹³¹I) can be obtained.

The compounds of general formula I or Ia comprising iodine radioisotopes (¹²³I, ¹²⁴I, ¹²⁵I, ¹³¹I) are particularly suitable for use in radiodiagnostics and radiotherapy. The compounds of general formula I or Ia comprising stable iodine isotope ¹²⁷I are particularly suitable for use as precursors of these radiopharmaceuticals and radiodiagnostics, or as medicaments.

The preparation of a radiopharmaceutical according to this invention is substantially facilitated by the fact that a non-radioactive pharmaceutical comprising ¹²⁷I can be converted to radiolabeled pharmaceutical by a simple palladium-catalysed isotopic exchange. This isotopic exchange can take place also in aqueous media under mild conditions, and with short reaction times. Thus, the preparation of a radiopharmaceutical can be performed also directly at the clinical department in an easy and advantageous manner with employment of a radiopharmaceutical kit.

The novel iodinated choline derivatives according to the present invention exhibit a higher affinity to choline transporters than the currently medically utilized fluoromethylcholine (or [¹⁸F]fluoromethylcholine, respectively), and some of them even possess a higher affinity than the natural ligand of the transporters - choline (or [¹¹C]choline, respectively). The derivatives of the present invention are more advantageous than the currently used imaging agents. Moreover, they can also be applied in the therapy of disorders involving an increased expression of choline transporters in the target tissue.

### Brief Description of Drawings

Fig. 1 represents a radiochromatogram of compound 4 labeled with the radioactive isotope ¹²⁵I prepared by the procedure described in example 23.
Fig. 2 represents an example of an inhibition curve of choline as described in Biological Assays for Examples 1-40 (inhibition of the uptake of [³H]choline by non-radioactive choline).

### Examples of carrying out the Invention

The invention is further illustrated by the following examples, which should not be construed as limiting the claimed scope.

Chemical shifts in the NMR spectra are given in ppm.

The notation in the NMR spectra: s (singlet), d (doublet), t (triplet), m (multiplet), br (broad signal). Abbreviations in the infrared spectra (IR): sh (shoulder in the absorption band), br (broadened band). The following abbreviations describe the intensity of the bands in the IR spectra: vs (very strong), s (strong), m (medium), w (weak), vw (very weak).

NMR spectra were recorded on Bruker DPX 300 (300 MHz for ¹H, 75 MHz for ¹³C) and Bruker Avance-III 600 (600 MHz for ¹H, 150 MHz for ¹³C) NMR spectrometers (Bruker Daltonik, Germany). Elemental analyses for C, H, N were performed on the instrument PE 2400 Series II Analyzer (Perkin Elmer, USA). Elemental analyses for sulfur and iodine were performed on the X-ray fluorescence spectrometer SPECTRO iQ II (Spectro Analytical Instruments, Germany). For chromatographic analyses, HPLC chromatograph Dionex Ultimate 3000 (Dionex, USA) with in-line measurement of radioactivity (FlowStar LB 513 Radio Flow Detector, Berthold Technologies, Germany) and a reverse-phase column (Chromolith Performance RP-18e 100 × 4.6 mm, Merck, Germany) was used. As elution mixture acetonitrile and water with 0.1 % of trifluoroacetic acid was used at a flow rate of 4 mL min⁻¹. Infrared spectra were obtained on the spectrometer Specac MKII Golden Gate Single Reflection ATR System, with a diamond crystal and ray incident angle of 45°.

### List of abbreviations

- BCA: Bicinchoninic acid assay
- IR: Infrared spectrum
- NMR: Nuclear magnetic resonance
- eq.: Equivalent
- meq.: Milliequivalent (amount of substance multiplied by the ion charge)
- DMF: *N,N*-dimethylformamide
- DMSO: Dimethyl sulfoxide
- v/v: Volume to volume ratio
- MeOH: Methanol
- Et₂O: Diethyl ether
- EtOAc: Ethyl acetate
- Ms: Methanesulfonyl
- MsO⁻: Methanesulfonate anion
- M: mol. L⁻¹
- Tris: 2-Amino-2-hydroxymethyl-propane-1,3-diol
- Ts: 4-Methylbenzenesulfonyl
- TsO⁻: 4-Methylbenzenesulfonate anion
- m.p.: Melting point
- ATR: Attenuated total reflectance
- ESI: Electrospray ionization
- HRMS: High resolution mass spectrometry
- PBS: Phosphate buffered saline
- cps: Counts per second (number of registered radionuclide decays per second)
- HPLC: High-performance liquid chromatography
- TLC: Thin-layer chromatography

### I. Synthesis of compounds

General procedure for the preparation of compounds **1** to **40** using alkylating agents **A** to **T** and the corresponding amines:
Magnetic stirrer, a corresponding alkylating agent, and a corresponding amine were put into a flask. Unless otherwise indicated, DMF was used as a solvent. The flask was flushed with argon, sealed with a rubber septum, immersed into a heated oil bath, and the reaction mixture was stirred. The course of the reaction was monitored by TLC. The solvent was subsequently removed *in vacuo* by co-evaporation with toluene. Unless otherwise indicated, the product was purified by re-precipitation, which was done as follows: the evaporated residue was dissolved in a minimal quantity of MeOH, transferred into a centrifugation tube, precipitated with a mixture of Et₂O and hexane, or with pure Et₂O, and the resulting suspension was centrifuged and decanted. The product was then dissolved in MeOH, transferred to a 25 mL pear-shaped flask using a Pasteur pipette, and the solvent was subsequently removed on a rotary evaporator by co-evaporation with toluene. The residual volatiles were finally removed under high vacuum.

Formulae of alkylating agents:

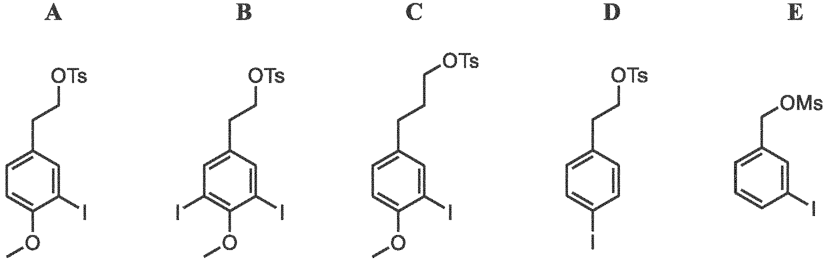

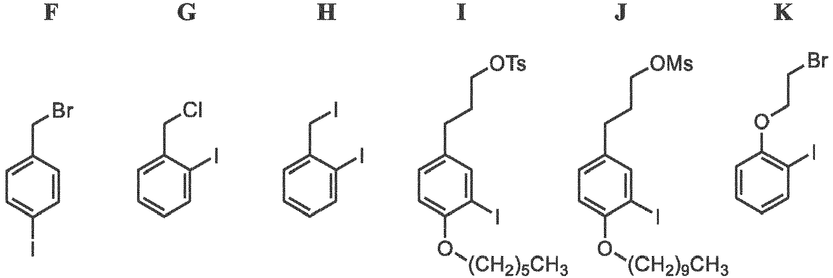

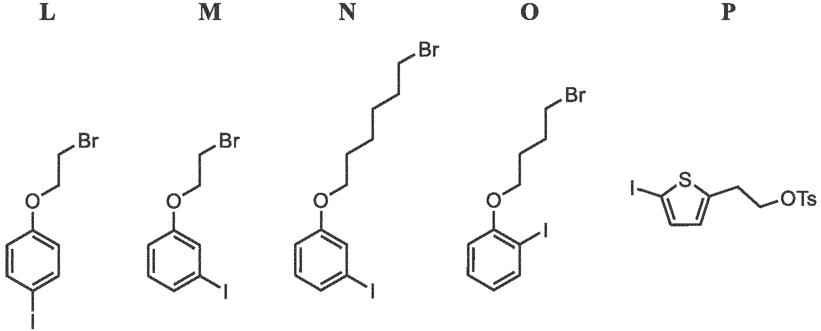

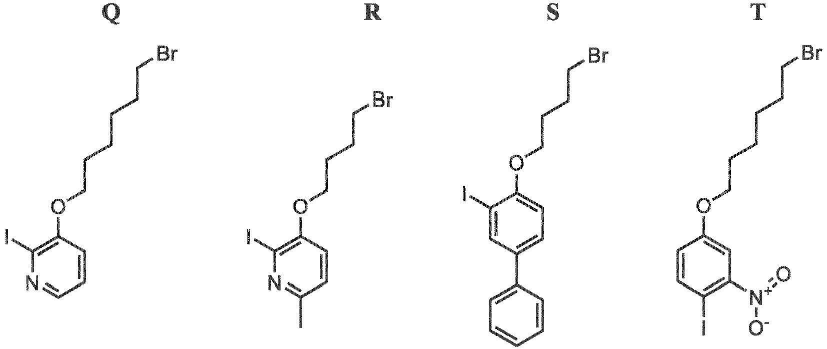

### Example 1

### Preparation of (2-hydroxyethyl)-[2-(3-iodo-4-methoxyphenyl)ethyl]-dimethylammonium 4-methylbenzenesulfonate (1)

The alkylating agent A (200 mg, 0.463 mmol, 1 eq.), DMF (2 mL), and 2-(dimethylamino)ethanol (140 µL, 1 .39 mmol, 3 eq.) were used for the synthesis of compound 1. The reaction time was 3 h at the temperature of 80 °C. The precipitation was done twice using a mixture of Et₂O and hexane (1:1 v/v, 45 mL). The product was obtained as a white solid (137 mg, 0.263 mmol, 57 %). 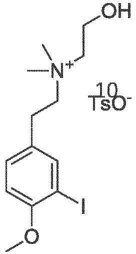 ¹H NMR (600 MHz, DMSO-*d*₆) δ 7.75 (d, *J*= 1.9 Hz, 1H), 7.48 (d, *J*= 8.0 Hz, 2H), 7.30 (dd, *J* = 8.4, 1.8 Hz, 1H), 7.11 (d, *J* = 7.9 Hz, 2H), 6.97 (d, *J* = 8.4 Hz, 1H), 5.34 (t, *J* = 5.0Hz, 1H), 3.88 - 3.83 (m, 2H), 3.80 (s, 3H), 3.52 - 3.47 (m, 2H), 3.48 - 3.44 (m, 2H), 3.13 (s, 6H), 2.98 - 2.94 (m, 2H), 2.28 (s, 3H). ¹³C NMR (151 MHz, DMSO-*d₆)* δ 156.76, 145.69, 139.27, 137.65, 130.47, 130.43, 128.09, 125.49, 111.62, 86.24, 64.82, 64.68, 56.42, 55.04, 50.87, 26.73, 20.82. IR (diamond-ATR): ν̃ (cm⁻¹) 3300m,br, 3081vw, 3036vw, 3006vw, 2946w, 2918vw, 2867vw, 2840vw, 1981vw, 1912vw, 1598w, 1566vw, 1491m, 1461m, 1439m, 1407w, 1394w, 1356w, 1280m, 1254m, 1217s, 1194s, 1178s, 1170s, 1119s, 1102m, 1052m, 1032s, 1018m, 1008s, 939w, 923m, 895w, 882w, 856w, 821s, 798w, 780w, 748w, 713w, 680s, 662m, 564s, 551s, 516w, 496w. HRMS (ESI) m/z: [M⁺] (C₁₃H₂₁INO₂⁺) calculated: 350.061148, found: 350.06124. Elem. anal. C₂₁H₂₈INO₄S, calculated: C (46.07), H (5.41), N (2.69), S (6.15), found: C (46.02), H (5.48), N (2.58), S (6.24).

### Example 2

### Preparation of 3-hydroxy-1-[2-(3-iodo-4-methoxyphenyl)ethyl]quinuclidin-1-ium 4-methylbenzenesulfonate (2) - not part of the invention

The alkylating agent **A** (200 mg, 0.463 mmol, 1 eq.), DMF (2 mL), and quinuclidin-3-ol (178 mg, 1.400 mmol, 3 eq.) were used for the synthesis of compound **2**. The reaction time was 3 h at the temperature of 80 °C. The precipitation was done first using a mixture of Et₂O and hexane (1:1 v/v, 45 mL), and subsequently with pure Et₂O (45 mL). The product was obtained as an off-white solid (238 mg, 0.425 mmol, 92 %). 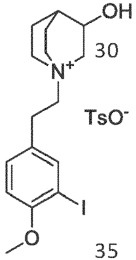 M.p. 173-176 °C. ¹H NMR(600 MHz, DMSO-d₆) δ 7.75 (d, *J* = 2.2 Hz, 1H), 7.48 (d, *J* = 8.0 Hz, 2H), 7.29 (dd, *J* = 8.4, 2.2 Hz, 1H), 7.11 (d, *J* = 7.9 Hz, 2H), 6.97 (d, *J* = 8.5 Hz, 1H), 5.60 (d, *J* = 3.5 Hz, 1H), 4.11 - 4.06 (m, 1H), 3.80 (s, 3H), 3.68 (ddd, *J* = 12.7, 8.2, 2.9 Hz, 1H), 3.47 - 3.41 (m, 1H), 3.40 - 3.27 (m, 5H), 3.09 (dt, *J* = 12.8, 2.8 Hz, 1H), 2.96 - 2.86 (m, 2H), 2.28 (s, 3H), 2.16 - 2.10 (m, 1H), 2.06 - 2.02 (m, 1H), 1.96 - 1.89 (m, 1H), 1.81 - 1.71 (m, 2H). ¹³C NMR (151 MHz, DMSO-*d₆*) δ 156.73, 145.73, 139.24, 137.62, 130.61, 130.43, 128.08, 125.49, 111.58, 86.22, 63.44, 62.65, 56.42, 54.03, 52.71, 26.36, 26.05, 20.90, 20.81, 17.39. IR (diamond-ATR): ν̃ (cm⁻¹) 3274m,br, 3014w, 2938w, 2918w, 2885w, 2840w, 1662vw, 1600w, 1570vw, 1491m, 1461w, 1421w, 1392w, 1363w, 1338w, 1324w, 1284w, 1254m, 1212s, 1180s, 1168s, 1119s, 1098m.1049m_{.}, 1032m, 1008s, 975w, 946w, 923w, 906w, 884w, 820m, 761w, 710w, 682s, 664m, 638w, 563s, 492w, 468w. HRMS (ESI) m/z: [M⁺] (C₁₆H₂₃INO₂⁺) calculated: 388.076798, found: 388.07672. Elem. anal. C₂₃H₃₀INO₅S, calculated: C (49.38), H (5.40), N (2.50), I (22.68), S (5.73), found: C (49.51) H (5.35), N (2.24), I (22.40), S (5.76).

### Example 3

### Preparation of (2-hydroxyethyl)-[2-(3,5-diiodo-4-methoxyphenyl)ethyl]-dimethylammonium 4-methylbenzenesulfonate (3)

The alkylating agent **B** (350 mg, 0.627 mmol, 1 eq.), DMF (3 mL), and 2-(dimethylamino)ethanol (190 µL, 1.888 mmol, 3 eq.) were used for the synthesis of compound **3**. The reaction time was 4 h at the temperature of 85 °C. The precipitation was done once using a mixture of Et₂O and hexane (1:1 v/v, 45 mL), and subsequently with pure Et₂O (40 mL). The product was obtained as a white solid (258 mg, 0.399 mmol, 64 %). 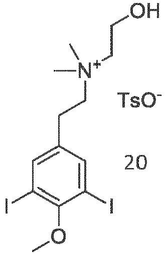 M.p. 178-180 °C, ¹H NMR (600 MHz, DMSO-*d*₆) δ 7.82 (s, 2H), 7.48 (d, *J* = 8.1 Hz, 2H), 7.11 (d, *J* = 7.9 Hz, 2H), 5.34 (t, *J* = 5.0 Hz, 1H), 3.88 - 3.83 (m, 2H), 3.73 (s, 3H), 3.52 - 3.48 (m, 2H), 3.46 - 3.43 (m, 2H), 3.11 (s, 6H), 3.00 - 2.93 (m, 2H), 2.28 (s, 3H). ¹³C NMR (151 MHz, DMSO-*d*₆) δ 157.38, 145.67, 140.12, 137.64, 136.74, 128.08, 125.48, 91.52, 64.94, 64.21, 60.29, 55.00, 50.90, 26.26, 20.82. IR (diamond-ATR): ν̃ (cm⁻¹) 3322m,br, 3032w, 2999vw, 2948vw, 2934vw, 2915vw, 2870vw, 2850vw, 2817vw, 1991vw, 1980vw, 1930vw, 1911vw, 1893vw, 1803vw, 1752vw, 1599vw, 1581w, 1536w, 1493w, 1480w, 1461m, 1414m, 1396w, 1377w, 1360w, 1337w, 1326w, 1306w, 1248m, 1207m, 1196s, 1173s, 1150m, 1122s, 1102m, 1094m, 1054w, 1032s, 1009s, 996s, 978w, 961w, 938m, 911w, 889w, 869w, 854w, 840w, 812m, 799w, 772w, 742w, 706m, 682s, 622w, 610w, 564s, 552s, 517w, 493w, 463w. HRMS (ESI) m/z: [M⁺] (C₁₃H₂₀I₂NO₂⁺) calculated: 475.957791, found: 475.95731, Elem. anal. C₂₀H₂₇I₂NO₅S, calculated: C (37.11), H (4.20), N (2.16), I (39.21), S (4.95), found: C (37.14), H (4.27), N (1.98), I (39.23), S (5.17).

### Example 4

### Preparation of (2-hydroxyethyl)-[3-(3-iodo-4-methoxyphenyl)propyl]-dimethylammonium 4-methylbenzenesulfonate (4)

The alkylating agent **C** was used (342 mg, 0.766 mmol, 1 eq.), DMF (3 mL), and 2-(dimethylamino)ethanol (190 µL, 1.888 mmol, 3 eq.) were used for the synthesis of compound **4**. The reaction time was 2 h at the temperature of 80 °C. The product was purified by chromatography on C₁₈-silica gel (eluent 4:6 MeOH:water v/v). The product was obtained as a white solid (354 mg, 0.661 mmol, 86 %). 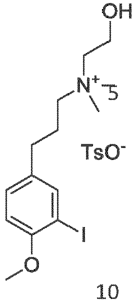 M.p. 120-122 °C. ¹H NMR (600 MHz, DMSO-*d*₆) δ 7.67 (d, *J* = 2.2 Hz, 1H), 7.49 (d, *J* = 8.0 Hz, 2H), 7.23 (dd, *J* = 8.4, 2.2 Hz, 1H), 7.12 (d, *J* = 7.9 Hz, 2H), 6.93 (d, *J* = 8.5 Hz, 1H), 5.28 (br s, 1H), 3.82 - 3.78 (m, 2H), 3.79 (s, 3H), 3.40 - 3.36 (m, 2H), 3.34 - 3.30 (m, 2H), 3.05 (s, 6H), 2.49 (t, *J* = 8.0 Hz, 2H), 2.28 (s, 3H), 1.98 - 1.92 (m, 2H). ¹³C NMR (151 MHz, DMSO-*d*₆) δ 156.22, 145.70, 138.53, 137.66, 134.67, 129.62, 128.10, 125.49, 111.44, 86.14, 64.63, 63.60, 56.37, 54.97, 50.91, 30.23, 23.86, 20.82. IR (diamond-ATR): ν̃ (cm⁻¹) 3324m,br, 3035vw, 3021vw, 3000vw, 2972vw, 2958vw, 2937w, 2862vw, 2840vw, 1981vw, 1915vw, 1655vw, 1598w, 1562vw, 1486m, 1474w, 1459w, 1441w, 1418w, 1400w, 1362w, 1290w, 1278w, 1253m, 1227m, 1214m, 1173s, 1150m, 1118s, 1096m, 1073m, 1048m, 1030s, 1020w, 1008s, 968w, 944w, 893w, 846w, 814s, 800w, 756w, 712w, 680s, 663m, 598w, 562s, 540m, 512w, 494w. HRMS (ESI) m/z: [M)⁺] (C₁₄H₂₃INO₂⁺) calculated: 364.076798, found: 364.07690. Elem. anal. C₂₁H₃₀INO₅S, calculated: C (47.11), H (5.65), N (2.62), S (5.99), found: C (47.18), H (5.63), N, (2.47), S (6.04).

### Example 5

### Preparation of (2-hydroxyethyl)-[2-(4-iodophenyl)ethyl]-dimethylammonium 4-methylbenzenesulfonate (5)

The alkylating agent **D** was used (400 mg, 0.99 mmol, 1 eq.), DMF (5 mL), and 2-(dimethylamino)ethanol (300 µL, 2.98 mmol, 3 eq.) were used for the synthesis of compound **5**. The reaction time was 2 h at the temperature of 60 °C and 2 h at the temperature of 80 °C. The precipitation was done once using the mixture of Et₂O and hexane (1:1 v/v, 45 mL) and subsequently twice with pure Et₂O (40 mL). The product was obtained as a white solid (270 mg, 0.55 mmol, 56 %). 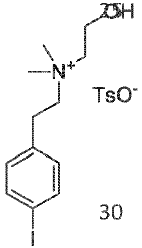 M.p. 204-206 °C. ¹H NMR (600 MHz, DMSO-*d*₆,) δ 7.70 (d, *J* = 8.2 Hz, 2H), 7.48 (d, *J =* 8.0 Hz, 2H), 7.13 (d, *J* = 8.2 Hz, 2H), 7.11 (d, *J* = 8.0 Hz, 2H), 5.35 (t, *J =* 5.0 Hz, 1H), 3.88 - 3.83 (m, 2H), 3.55 - 3.50 (m, 2H), 3.49 - 3.44 (m, 2H), 3.13 (s, 6H), 3.03 - 2.97 (m, 2H), 2.28 (s, 3H). ¹³C NMR (151 MHz, DMSO-*d*₆) δ 145.66, 137.67, 137.38, 136.25, 131.48, 128.10, 125.48, 92.88, 64.78, 64.27, 55.04, 50.90, 27.73, 20.82. IR (diamond-ATR): ν̃ (cm⁻¹) 3347m,br, 3041w, 3026w, 3003w, 2968w, 2947w, 2915w, 2864w, 1981vw, 1936vw, 1902vw, 1596w, 1484m, 1468w, 1442w, 1424w, 1406w, 1400w, 1354w, 1322w, 1306w, 1287w, 1218s, 1181s, 1168s, 1154m, 1118s, 1102m, 1084w, 1063m, 1053w, 1030s, 1007s, 975w, 946w, 923m, 907w, 883w, 858w, 832m, 824m, 802m, 730w, 713w, 682s, 628w, 564s, 552s, 512m, 453w. HRMS (ESI) m/z: [M⁺] (C₁₂H₁₉INO⁺) calculated: 320.050584, found: 320.05048. Elem. anal. C₁₉H₂₆INO₄S. calculated: C (46.44), H (5.33), N (2.85), I (25.83), S (6.53), found: C (46.65), H (5.32), N (2.64), I (25.56), S (6.55).

### Example 6

### Preparation of 3-hydroxy-1-[2-(4-iodophenyl)ethyl]-1-methylpiperidin-1-ium 4-methylbenzenesulfonate (6) ― not part of the invention

The alkylating agent **D** (400 mg, 0.99 mmol, 1 eq.), DMF (5 mL), and 1-methylpiperidine-3-ol (344 µL, 2.98 mmol, 3 eq.) were used for the synthesis of compound 6. The reaction time was 2 h at the temperature of 60 °C, 2 h at the temperature of 80 °C, and 2 h at the temperature of 90 °C.

The precipitation was done three times using a mixture of Et₂O and hexane (1:1 v/v, 45 mL). The product was obtained as a mixture of two diastereomers (1 : 1 ratio according to the NMR analysis) as a pale-yellow solid(269 mg, 0.52 mmol, 53 %). 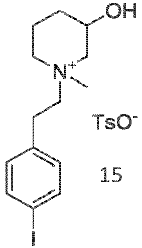 ¹H NMR (600 MHz, DMSO-*d*₆) δ 7.71 (d, *J* = 8.3 Hz, 1H, diastereomer 1), 7.70 (d, *J* = 8.3 Hz, 1H, diastereomer 2), 7.48 (d, *J* = 8.2 Hz, 2H, 4-methylbenzenesulfonate), 7.16 (d, *J* = 8.2 Hz, 1H), 7.13 (d, *J* = 8.2 Hz, 1H), 7.11 (d, *J* = 8.2 Hz, 2H, 4-methylbenzenesulfonate), 5.52 (d, *J* = 4.5 Hz, 1H), 5.44 (d, *J* = 4.2 Hz, 1H), 4.06 ― 3.99 (m, 2H), 3.72 ― 3.66 (m, 1H), 3.58 ― 3.49 (m, 2H), 3.48 ― 3.30 (m, 6H), 3.21 (s, 3H), 3.16 (d, *J* = 7.0 Hz, 1H), 3.14 (d, *J* = 6.9 Hz, 1H), 3.10 (s, 3H), 3.07 ― 2.94 (m, 4H), 2.28 (s, 6H), 1.98 ― 1.87 (m, 2H), 1.87 ― 1.75 (m, 4H), 1.52 - 1.40 (m, 2H). ¹³C NMR(151 MHz, DMSO-*d*₆) δ 145.70, 137.64, 137.41, 137.34, 136.37, 136.19, 131.58, 131.50, 128.09, 125.49, 92.89, 92.88, 65.02, 63.55, 63.03, 62.78, 61.45, 61.31, 60.02, 59.84, 49.93, 47.83, 30.00, 29.38, 27.24, 26.93, 20.82, 17.06, 16.70.IR (diamond-ATR): ṽ (cm⁻¹) 3320m,br, 3056vw, 3021vw, 2998vw, 2949w, 2922vw, 2869vw, 1979vw, 1968vw, 1954vw, 1933vw, 1915vw, 1906vw, 1598w, 1585w, 1562w, 1493w, 1457m, 1383w, 1354w, 1278w, 1206m, 1176s, 1119s, 1080m, 1052w, 1032s, 1008s, 914w, 876m, 816m, 765m, 745w, 734w, 711w, 679s, 648m, 563s, 518m, 494m. HRMS (ESI) m/z: [M⁺] (C₁₄H₂₁INO⁺) calculated: 346.066234, found: 346.06600, Elem. anal. C₂₁H₂₈INO₄S, calculated: C (48.75), H (5.45), N (2.71), I (24.53), S (6.20), found: C (48.91), H (5.48), N (2.59), I (24.25), S (6.25).

### Example 7

### Preparation of 1-(2-hydroxyethyl)-1-[2-(4-iodophenyl)ethyl]pyrrolidin-1-ium 4-methylbenzenesulfonate (7)

The alkylating agent **D** (400 mg, 0.99 mmol, 1 eq.), DMF (5 mL), and 2-(pyrrolidin-1-yl)ethanol (350 µL, 2.99 mmol, 3 eq.) were used for the synthesis of compound **7**. The reaction time was 2 h at the temperature of 60 °C, 2 h at the temperature of 80 °C, and 2 h at the temperature of 90 °C.

The precipitation was done three-times using a mixture of Et₂O and hexane (1:2 v/v, 45 mL). The product was obtained in as a pale-yellow solid (165 mg, 0.32 mmol, 32 %). 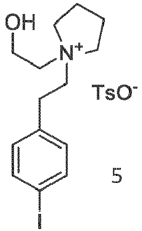 M.p. 152-154 °C. ¹H NMR (600 MHz, DMSO-*d*₆) δ 7.69 (d, *J* = 7.9 Hz, 2H), 7.48 (d, *J* = 7.8 Hz, 2H), 7.15 (d, *J* = 7.9 Hz, 2H), 7.11 (d, *J* = 7.8 Hz, 2H), 5.40 (t, *J* = 5.0 Hz, 1H), 3.88 ― 3.83 (m, 2H), 3.66 ― 3.54 (m, 4H), 3.52 ― 3.46 (m, 4H), 3.04 ― 2.99 (m, 2H), 2.28 (s, 3H), 2.10 ― 2.02 (m, 4H). ¹³C NMR (151 MHz, DMSO-*d*₆) δ 145.67, 137.66, 137.32, 136.36, 131.51, 128.09, 125.48, 92.84, 62.78, 60.27, 60.08, 55.73, 28.36, 21.21, 20.82. IR (diamond-ATR): ṽ (cm⁻¹) 3314m,br, 3064vw, 3040vw, 3021vw, 3007vw, 2978vw, 2965vw, 2916vw, 2890vw, 2862vw, 1995vw, 1981vw, 1935vw, 1912vw, 1823vw, 1795vw, 1670vw, 1598w, 1484m, 1468w, 1443w, 1418w, 1402w, 1389w, 1379w, 1359w, 1346w, 1321w, 1307w, 1212s, 1200m, 1184s, 1168s, 1120s, 1104m, 1086m, 1060w, 1032s, 1007s, 973w, 941w, 908w, 884w, 835m, 822m, 800m, 724w, 712w, 682m, 655m, 641m, 562s, 553s,sh, 524w, 508m, 496w. HRMS (ESI) m/z: [M)⁺] (C₁₄H₂₁INO⁺) calculated: 346.066234, found: 346.06629, Elem. anal. C₂₁H₂₈INO₄S, calculated: C (48.75), H (5.45), N (2.71), I (24.53), S (6.20), found: C (48.93), H (5.46), N (2.53), I (24.07), S (6.30).

### Example 8

### Preparation of 3-hydroxy-1-[2-(4-iodophenyl)ethyl]quinuclidin-1-ium 4-methylbenzenesulfonate (8) ― not part of the invention

The alkylating agent **D** (400 mg, 0.99 mmol, 1 eq.), DMF (5 mL), and quinuclidin-3-ol (380 mg, 2.99 mmol, 3 eq.) were used for the synthesis of compound **8**. The reaction time was 3 h at the temperature of 60 °C. The precipitation was done once using a mixture of Et₂O and hexane (1:1 v/v, 45 mL), and twice with pure Et₂O (40 mL). The product was obtained as an off-white solid (425 mg, 0.80 mmol, 80 %). 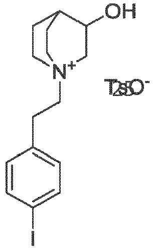 M.p. 217-218 °C. ¹H NMR (600 MHz, DMSO-*d*₆) δ 7.70 (d, *J* = 8.2 Hz, 2H), 7.48 (d, *J* = 8.0 Hz, 2H), 7.14 ― 7.10 (m, 4H), 5.60 (d, *J* = 3.5 Hz, 1H), 4.10 ― 4.06 (m, 1H), 3.69 (ddd, *J* = 12.7, 8.3, 3.0 Hz, 1H), 3.48 ― 3.42 (m, 1H), 3.41 ― 3.28 (m, 5H), 3.09 (dt, *J* = 12.9, 2.8 Hz, 1H), 2.99 ― 2.90 (m, 2H), 2.28 (s, 3H), 2.17 ― 2.09 (m, 1H), 2.06 ― 2.01 (m, 1H), 1.95 ― 1.88 (m, 1H), 1.81 ― 1.70 (m, 2H). ¹³C NMR (151 MHz, DMSO-*d*₆) δ 145.69, 137.65, 137.35, 136.43, 131.46, 128.09, 125.49, 92.83, 63.44, 63.02, 62.67, 54.04, 52.74, 27.06, 26.36, 20.90, 20.82, 17.39. IR (diamond-ATR): ṽ (cm⁻¹) 3259m,br, 3061vw, 3036vw, 3020vw, 3011vw, 2956w, 2920vw, 2879vw, 1991vw, 1980vw, 1920vw, 1899vw, 1802vw, 1655vw, 1596vw, 1486m, 1456w, 1386w, 1360w, 1338w, 1323w, 1306w, 1217s, 1184s, 1137w, 1120s, 1096m, 1060m, 1031s, 1006s, 969w, 954w, 944w, 922w, 906w, 884w, 841w, 816m, 806m,sh, 720w, 711w, 680s, 639w, 564s, 551m,sh, 508m, 485w. HRMS (ESI) m/z: [M⁺] (C₁₅H₂₁INO⁺) calculated: 358.066234, found: 358.06608, Elem. anal. C₂₂H₂₈INO₄S, calculated: C (49.91), H (5.33), N (2.65), I (23.97), S (6.06), found: C (50.12), H (5.37), N (2.46), I (23.73), S (6.21).

### Example 9

### Preparation of (2-hydroxyethyl)-(3-iodobenzyl)-dimethylammonium methanesulfonate (9)

The alkylating agent **E** (200 mg, 0.67 mmol, 1 eq.), DMF (1.5 mL), and 2-(dimethylamino)ethanol (134 µL, 1.33 mmol, 2 eq.) were used for the synthesis of compound **9**. The reaction time was 1 h at the temperature of 80 °C. The the product was precipitated once from a dichloromethane solution with a mixture of Et₂O and hexane (2:1 v/v, 45 mL) and once from MeOH solution by a mixture of Et₂O and hexane (2:1 v/v, 45 mL). The product was obtained in as a colorless viscous liquid (259 mg, 0.67 mmol, 100 %). 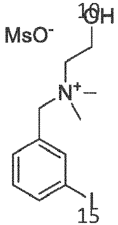 ¹H NMR (300 MHz, DMSO-*d*₆) δ 7.99 (dd, *J* = 1.8, 1.7 Hz, 1H), 7.91 (ddd, *J* = 7.9, 1.8, 1.1 Hz, 1H), 7.60 (ddd, *J* = 7.7, 1.7, 1.1 Hz, 1H), 7.32 (dd, *J* = 7.9, 7.7 Hz, 1H), 5.36 (t, *J* = 4.9 Hz, 1H), 4.53 (s, 2H), 3.95 ― 3.87 (m, 2H), 3.41 ― 3.34 (m, 2H), 3.01 (s, 6H), 2.30 (s, 3H).

### Example 10

### Preparation of 1-(2-hydroxyethyl)-1-(3-iodobenzyl)pyrrolidin-1-ium methanesulfonate (10)

The alkylating agent **E** (400 mg, 1.27 mmol, 1 eq.), DMF (2.0 mL), and 2-(pyrrolidin-1-yl)ethanol (223 µL, 1.91 mmol, 1.5 eq.) were used for the synthesis of compound **10**. The reaction time was 1 h at the temperature of 75 °C. The product was precipitated from a MeOH solution once with a mixture of Et₂O and hexane (2:1 v/v, 45 mL) and once with pure Et₂O (45 mL). The product was obtained as a yellowish viscous liquid (500 mg, 1.16 mmol, 91 %). 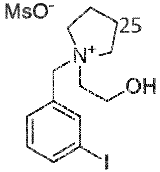 ¹H NMR (300 MHz, DMSO-*d*₆) δ 8.02 (t, *J* = 1.7 Hz, 1H), 7.89 (dt, *J* = 7.9, 1.1 Hz, 1H), 7.65 (dt, *J* = 7.7, 1.2 Hz, 1H), 7.31 (t, *J* = 7.8Hz, 1H), 5.51 (t, *J* = 4.8Hz, 1H), 4.61 (s, 2H), 3.94 (dt, *J* = 5.0, 4.8 Hz, 2H), 3.64 ― 3.44 (m, 4H), 3.32 ― 3.25 (m, 2H), 2.34 (s, 3H), 2.11 ― 2.03 (m, 4H). ¹³C NMR (75 MHz, DMSO-*d*₆) δ 141.04, 138.83, 132.29, 131.34, 130.95, 95.30, 61.67, 60.97, 59.94, 55.40, 54.94, 20.84.

### Example 11

### Preparation of (2-hydroxyethyl)-(4-iodobenzyl)-dimethylammonium bromide (11)

The alkylating agent **F** (400 mg, 1.35 mmol, 1 eq.), DMF (2 mL), and 2-(dimethylamino)ethanol (303 µL, 3.01 mmol, 2.2 eq.) were used for the synthesis of compound **11**. The reaction time was 30 min at the temperature of 75 °C. The from the product was precipitated from a MeOH solution once with a mixture of Et₂O and hexane (3:1 v/v, 40 mL) and once with pure Et₂O (40 mL). The product was obtained as a white solid (472 mg, 1.22 mmol, 91 %). 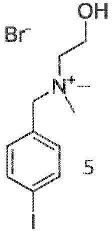 ¹H NMR (300 MHz, DMSO-*d*₆) δ 7.88 (d, *J* = 8.2 Hz, 2H), 7.39 (d, *J* = 8.2 Hz, 2H), 5.34 (t, *J* = 5.0 Hz, 1H), 4.63 (s, 2H), 3.94 ― 3.86 (m, 2H), 3.43 ― 3.37 (m, 2H), 3.03 (s, 6H). ¹³C NMR (75 MHz, DMSO-*d*₆) δ 137.67, 135.10, 127.75, 98.01, 66.51, 64.88, 54.83, 49.78.

### Example 12

### Preparation of diethyl-(2-hydroxyethyl)-(4-iodobenzyl)ammonium bromide (12)

The alkylating agent **F** (400 mg, 1.35 mmol, 1 eq.), DMF (2 mL), and 2-(diethylamino)ethanol (268 µL, 2.02 mmol, 1.5 eq.) were used for the synthesis of compound **12**. The reaction time was 30 min at the temperature of 75 °C. The precipitation was performed from a MeOH solution once with the mixture of Et₂O and hexane (3:1 v/v, 40 mL) and once with pure Et₂O (40 mL). The product was obtained as a white solid (459 mg, 1.11 mmol, 82 %). 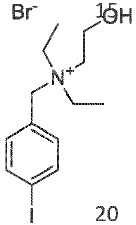 ¹H NMR (300 MHz, DMSO-*d*₆) δ 7.87 (d, *J* = 8.3 Hz, 2H), 7.36 (d, *J* = 8.3 Hz, 2H), 5.38 (t, *J* = 5.1 Hz, 1H), 4.58 (s, 2H), 3.88 (dt, *J* = 5.2, 5.1 Hz, 1H), 3.30 ― 3.20 (m, 6H), 1.31 (t, *J* = 7.1 Hz, 6H). ¹³C NMR (75 MHz, DMSO-*d*₆) δ 137.76, 134.87, 127.48, 97.87, 60.30, 58.06, 54.45, 53.25, 7.79.

### Example 13

### Preparation of 1-(2-hydroxyethyl)-1-(4-iodobenzyl)pyrrolidin-1-ium bromide (13)

The alkylating agent **F** (400 mg, 1.35 mmol, 1 eq.), DMF (2 mL), and 2-(pyrrolidin-1-yl)ethanol (236 µL, 2.02 mmol, 1.5 eq.) were used for the synthesis of compound **13**. The reaction time was 30 min at the temperature of 75 °C. The product was precipitated from a MeOH solution once with a mixture of Et₂O and hexane (3:1 v/v, 40 mL) and once with pure Et₂O (40 mL). The product was obtained as a white solid(484 mg, 1.17 mmol, 87 %). 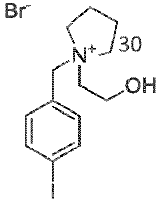 ¹H NMR (300 MHz, DMSO-*d*₆) δ 7.87 (d, *J* = 8.3 Hz, 2H), 7.42 (d, *J* = 8.3 Hz, 2H), 5.43 (t, *J* = 4.9 Hz, 1H), 4.63 (s, 2H), 3.93 (dt, *J* = 4.9, 4.9 Hz, 2H), 3.64 ― 3.45 (m, 4H), 3.31 ― 3.23 (m, 2H), 2.14 ― 1.99 (m, 4H). ¹³C NMR (75 MHz, DMSO-*d*₆) δ 137.75, 134.86, 128.42, 97.83, 61.71, 60.90, 59.75, 55.31, 20.78.

### Example 14

### Preparation of 1-(2-hydroxyethyl)-1-(2-iodobenzyl)piperidin-1-ium 4-methylbenzenesulfonate (14)

The alkylating agent **G** (600 mg, 2.38 mmol, 1 eq.) and 1-(2-hydroxyethyl)piperidine (4 mL, 30 mmol, 12.5 eq.) without any additional solvent were used for the synthesis of compound **14**. The reaction was carried out for 18 h at the temperature of 80 °C. The reaction mixture was diluted with water (50 mL) and the residual amine was removed by extraction with ethyl acetate (4 × 40 mL). The aqueous phase was concentrated, the oily residue was dissolved in MeOH and was slowly passed through a column containing a strong anion exchange resin (30 mL, particle size 650 - 820 µm, 1 mekv. mL⁻¹) in TsO⁻ cycle. The solvent from the collected solution was removed *in vacuo.*The product was obtained in the form of brown viscous liquid, which subsequently crystallized upon cooling (938 mg, 1.81 mmol, 76%). 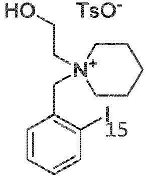 ¹H NMR (300 MHz, DMSO-*d*₆) δ 8.08 (dd, *J* = 8.0, 1.3 Hz, 1H), 7.68 (dd, *J* = 7.8, 1.6 Hz, 1H), 7.54 (ddd, *J* = 7.5, 7.5, 1.3 Hz, 1H), 7.49 (d, *J* = 8.1 Hz, 2H), 7.26 (ddd, *J* = 7.8, 7.8, 1.5 Hz, 1H), 7.11 (d, *J* = 7.9 Hz, 2H), 5.48 (t, *J* = 4.7 Hz, 1H), 4.80 (s, 2H), 4.08 ― 3.87 (m, 2H), 3.73 ― 3.58 (m, 4H), 3.19 ― 3.07 (m, 2H), 2.28 (s, 3H), 2.05 ― 1.83 (m, 2H), 1.82 ― 1.67 (m, 2H), 1.65 ― 1.47 (m, 1H), 1.40 ― 1.17 (m, 1H). ¹³C NMR (75 MHz, DMSO-*d*₆) δ 145.66, 140.99, 137.65, 135.25, 132.16, 130.87, 128.79, 128.07, 125.48, 105.67, 69.34, 58.65, 57.77, 54.94, 20.80, 20.09, 19.29. IR (diamond-ATR): ṽ (cm⁻¹) 3343m,br, 3086vw, 3063vw, 3023w, 2960w, 2918vw, 2884w, 1982vw, 1967vw, 1953vw, 1916vw, 1660vw, 1598vw, 1586vw, 1492w, 1465w, 1455m, 1436w, 1400w, 1346w, 1315w, 1290w, 1213s, 1178s, 1120s, 1103m, 1052m, 1034s, 1010s, 990w, 946w, 932w, 908w, 868w, 818m, 798w, 763s, 726w, 712w, 679s, 650m, 623w, 561s, 464w. HRMS (ESI) m/z: [M⁺] (C₁₄H₂₁INO⁺) calculated: 346.066234, found: 346.06628, Elem. anal. C₂₁H₂₈INO₄S, calculated: C (48.75), H (5.45), N (2.71), S (6.20), found: C (49.07), H (5.58), N (2.62), S (6.28).

### General procedure for preparation of compounds 15 to 18 with the alkylating agent H:

A magnetic stir bar, alkylating agent **H** (400 mg, 1.16 mmol, 1 eq.), DMF (5 mL), and an appropriate amine (3.49 mmol, 3 eq.) were put into a pear-shaped flask. The flask was flushed with argon, closed by a rubber septum, immersed into an oil bath heated to 50 °C, and the reaction mixture was stirred for 2 h. The course of the reaction was monitored by TLC. The solvent was removed on a rotary evaporator by co-evaporation with toluene. The residue was then dissolved in a minimum amount of MeOH, transferred into a centrifugation tube, precipitated with a mixture of Et₂O and hexane (1:1 v/v, 45 mL), centrifuged and decanted. The resulting pellet was then re-dissolved in a minimal volume of MeOH, precipitated with pure Et₂O, centrifuged and decanted (this procedure was repeated two times). The obtained product was dissolved in MeOH and was passed slowly through a column containing a strong anion exchange resin (15 mL, particle size 650 - 820 µm, 1 meq. mL⁻¹) in TsO⁻ cycle. The solvent was removed from the solution on a rotary evaporator. In the case of liquid samples, solvent were removed by co-evaporation with toluene. Residual solvents were then removed under high vacuum.

### Example 15

### Preparation of Diethyl-(2-hydroxyethyl)-(2-iodobenzyl)ammonium 4-methylbenzenesulfonate (15)

A colorless viscous liquid, which crystallized upon cooling (477 mg, 0.94 mmol, 81 %). 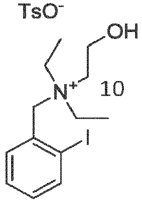 M.p. 103-105°C. ¹H NMR (600 MHz, DMSO-*d*₆) δ 8.07 (dd, *J* = 7.9, 1.3 Hz, 1H), 7.67 (dd, *J* = 7.8, 1.4 Hz, 1H), 7.55 (ddd, *J* = 7.6, 7.6, 1.3 Hz, 1H), 7.48 (d, *J* = 8.0 Hz, 2H), 7.26 (ddd, *J* = 7.7, 7.7, 1.6 Hz, 1H), 7.11 (d, *J* = 7.9 Hz, 2H), 5.44 (br s, 1H), 4.72 (s, 2H), 3.87 (br s, 2H), 3.47 ― 3.37 (m, 6H), 2.28 (s, 3H), 1.28 (t, *J* = 7.1 Hz, 6H). ¹³C NMR (151 MHz, DMSO-*d*₆) δ 145.71, 140.92, 137.63, 134.11, 132.17, 131.26, 129.07, 128.08, 125.49, 105.51, 65.31, 59.38, 54.76, 54.32, 20.81, 8.18. IR (diamond-ATR): ṽ (cm⁻¹) 3270m,br, 3010w, 2990vw, 2972w, 2916vw, 2889vw, 2870vw, 1990vw, 1980vw, 1646vw, 1596vw, 1581vw, 1567vw, 1494w, 1468w, 1457w, 1443w, 1424w, 1402w, 1382w, 1357w, 1304w, 1274w, 1215m, 1186s, 1160m, 1116m, 1090m, 1052w, 1033m, 1010s, 982w, 963w, 934w, 890w, 858w, 820m, 802w, 785w, 760m, 730w, 721m, 713m, 678s, 648m, 624w, 562s, 526w, 496w, 458w. HRMS (ESI) m/z: [M⁺] (C₁₃H₂₁INO⁺) calculated: 334.066234, found: 334.06648. Elem. anal. C₂₀H₂₈INO₄S, calculated: C (47.53), H (5.58), N (2.77), I (25.11), S (6.34), found: C (47.42), H (5.60), N (2.74), I (25.01), S (6.51).

### Example 16

### Preparation of 3-hydroxy-1-(2-iodobenzyl)-1-methylpiperidin-1-ium 4-methylbenzenesulfonate (16) ― not part of the invention

Mixture of diastereomers (2:1 ratio according to the NMR analysis) in the form of an off-white hygroscopic solid (424 mg, 0.84 mmol, 73 %). 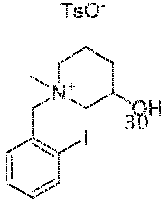 ¹H NMR (300 MHz, DMSO-*d*₆) δ 8.08 (dd, *J* = 8.0, 1.3 Hz, 1H), 7.72 (dd, *J* = 7.8, 1.7 Hz, 0.67H), 7.65 (dd, *J* = 7.7, 1.7 Hz, 0.33H), 7.58 ― 7.52 (m, 1H), 7.49 (d, *J* = 8.0 Hz, 2H), 7.27 (ddd, *J* = 7.7, 7.7, 1.7 Hz, 1H), 7.11 (d, *J* = 7.9 Hz, 2H), 5.50 (s, 1H), 4.84 ― 4.58 (m, 2H), 4.22 ― 4.14 (m, 0.33H), 4.05 ― 3.93 (m, 0.67H), 3.57 ― 3.01 (m, 4H), 3.14 (s, 1H), 3.04 (s, 2H), 2.29 (s, 3H), 2.11 ― 1.69 (m, 3H), 1.68 ― 1.52 (m, 0.33H), 1.41 ― 1.22 (m, 0.67H). ¹³C NMR (75 MHz, DMSO-*d*₆) δ 145.70, 145.69, 141.01, 137.61, 135.16, 134.84, 132.25, 132.23, 130.76, 130.71, 128.78, 128.72, 128.06, 125.48, 105.15, 105.10, 72.17, 63.77, 63.54, 61.94, 60.99, 60.45, 59.76, 49.23, 46.65, 30.51, 28.50, 20.79, 17.76, 17.75, 16.31.IR (diamond-ATR): ṽ (cm⁻¹) 3320m,br, 3056vw, 3021vw, 2998vw, 2949w, 2922vw, 2869vw, 1979vw, 1968vw, 1954vw, 1933vw, 1915vw, 1906vw, 1598w, 1585w, 1562w, 1493w, 1457m, 1383w, 1354w, 1278w, 1206m, 1176s, 1119s, 1080m, 1052w, 1032s, 1008s, 914w, 876m, 816m, 765m, 745w, 734w, 711w, 679s, 648m, 563s, 518m, 494m. HRMS (ESI) m/z: [M⁺] (C₁₃H₁₉INO⁺) calculated: 332.050584, found: 332.05079. Elem. anal. C₂₀H₂₆INO₄S·0.5H₂O, calculated: C (46.88), H (5.31), N (2.73), I (24.77), S (6.26), found: C (47.13), H (5.36), N (2.78), I (24.67).

### Example 17

### Preparation of 1,4-bis(2-hydroxyethyl)-1-(2-iodobenzyl)piperazin-1-ium 4-methylbenzenesulfonate (17)

White amorphous highly hygroscopic solid (502 mg, 0.89 mmol, 77 %). 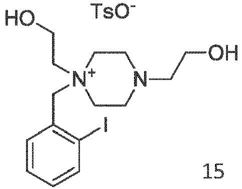 ¹H NMR (600 MHz, DMSO-*d*₆) δ 8.09 (dd, *J* = 8.0, 1.2 Hz, 1H), 7.71 (dd, *J* = 7.7, 1.6 Hz, 1H), 7.56 (ddd, *J* = 7.6, 7.6, 1.2 Hz, 1H), 7.49 (d, *J* = 8.0 Hz, 2H), 7.28 (ddd, *J* = 7.7, 7.7, 1.6 Hz, 1H), 7.12 (d, *J* = 7.9 Hz, 2H), 5.52 (t, *J* = 4.8 Hz, 1H), 4.86 (s, 2H), 4.49 (br s, 1H), 4.03 ― 3.99 (m, 2H), 3.77 ― 3.65 (m, 4H), 3.44 (q, *J =* 5.7 Hz, 2H), 3.24 (t, *J* = 10.7 Hz, 2H), 2.96 ― 2.86 (m, 2H), 2.82 ― 2.65 (m, 2H), 2.46 ― 2.41 (m, 2H), 2.28 (s, 3H). ¹³C NMR (151 MHz, DMSO-*d*₆) δ 145.64, 141.06, 137.70, 135.55, 132.33, 130.64, 128.85, 128.11, 125.50, 105.87, 69.36, 58.71, 58.47, 58.27, 56.81, 54.94, 46.05, 20.82. IR (diamond-ATR): ṽ (cm⁻¹) 3352m,br, 3082vw, 3056vw, 3018vw, 2937w, 2920w, 2869vw, 2834w, 1993vw, 1966vw, 1925vw, 1904vw, 1654vw, 1598w, 1584w, 1562w, 1494w, 1453m, 1360w, 1283w, 1207s, 1173s, 1120s, 1081w, 1053w, 1031s, 1008s, 988w, 944w, 932w, 888w, 855w, 816m, 800w, 764m, 728w, 711w, 680s, 648w, 633w, 601w, 563s, 493w, 450w. HRMS (ESI) m/z: [M⁺] (C₁₅H₂₄IN₂O₂⁺) calculated: 391.087697, found: 391.08771.

### Example 18

### Preparation of 3-hydroxy-1-(2-iodobenzyl)quinuclidin-1-ium 4-methylbenzenesulfonate (18) ― not part of the invention

White solid (496 mg, 0.96 mmol, 83 %). 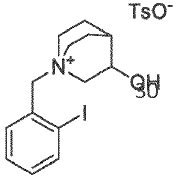 M.p. 154-156 °C. ¹H NMR (600 MHz, DMSO-*d*₆) δ 8.07 (dd, *J* = 8.0, 1.2 Hz, 1H), 7.66 (dd, *J* = 7.7, 1.6 Hz, 1H), 7.54 (ddd, *J* = 7.5, 7.5, 1.3 Hz, 1H), 7.48 (d, *J* = 8.1 Hz, 2H), 7.25 (ddd, *J* = 7.7, 7.7, 1.6 Hz, 1H), 7.11 (d, *J* = 7.9 Hz, 2H), 5.60 (s, 1H), 4.59 (d, *J* = 13.5 Hz, 1H), 4.54 (d, *J* = 13.5 Hz, 1H), 4.08 ― 4.02 (m, 1H), 3.74 (ddd, *J* = 12.7, 8.3, 3.0 Hz, 1H), 3.51 (tt, *J* = 11.2, 3.8 Hz, 1H), 3.47 ― 3.32 (m, 3H), 3.14 (dt, *J* = 12.7, 2.8 Hz, 1H), 2.28 (s, 3H), 2.15 ― 2.07 (m, 1H), 2.03 ― 1.99 (m, 1H), 1.94 ― 1.87 (m, 1H), 1.80 ― 1.70 (m, 2H). ¹³C NMR (151 MHz, DMSO-*d*₆) δ 145.73, 140.90, 137.63, 135.04, 132.17, 130.73, 128.78, 128.08, 125.50, 105.03, 69.40, 63.31, 63.21, 54.68, 53.51, 26.39, 20.97, 20.82, 17.47. IR (diamond-ATR): ṽ (cm⁻¹) 3185m,br, 3088w, 3060w, 3052w, 3013w, 2982w, 2946w, 2888w, 1931vw, 1855vw, 1826vw, 1664vw, 1598w, 1586w, 1560w, 1490w, 1460m, 1436w, 1425w, 1412w, 1385w, 1347w, 1318w, 1279w, 1217m, 1171s, 1133m,sh, 1119s, 1092m, 1070w, 1048w, 1032s, 1010s, 997m, 966w, 940w, 910w, 889w, 852w, 827m, 802w, 774m, 733w, 714w, 678s, 648w, 619w, 601w, 560s, 550m,sh, 484w, 469w. HRMS (ESI) m/z: [M⁺] (C₁₄H₁₉INO⁺) calculated: 344.050584, found: 344.05065. Elem. anal. C₂₁H₂₆INO₄S, calculated: C (48.94), H (5.08), N (2.72), I (24.62), S (6.22), found: C (48.64), H (5.15), N (2.59), I (24.21), S (6.11).

### Example 19

### Preparation of (2-hydroxyethyl)-(2-iodobenzyl)-dimethylammonium 4-methylbenzenesulfonate (19)

Colorless viscous liquid (500 mg, 1.05 mmol, 90%). 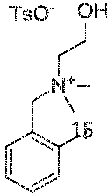 ¹H NMR (600 MHz, DMSO-*d*₆) δ 8.08 (dd, *J* = 8.0, 1.2 Hz, 1H), 7.72 (dd, *J* = 7.7, 1.6 Hz, 1H), 7.55 (ddd, *J* = 7.5, 7.5, 1.3 Hz, 1H), 7.48 (d, *J* = 8.0 Hz, 2H), 7.27 (ddd, *J*= 7.7, 7.7, 1.6 Hz, 1H), 7.11 (d, *J* = 7.8 Hz, 2H), 5.41 (br s, 1H), 4.72 (s, 2H), 3.97 ― 3.91 (m, 2H), 3.56 ― 3.53 (m, 2H), 3.09 (s, 6H), 2.28 (s, 3H). ¹³C NMR (151 MHz, DMSO-*d*₆) δ 145.71, 140.97, 137.64, 134.94, 132.28, 131.16, 128.82, 128.09, 125.50, 105.11, 70.48, 66.54, 55.15, 50.31, 20.82. IR (diamond-ATR): ṽ (cm⁻¹) 3321m,br, 3082w, 3046w, 3024w, 2965w, 2919w, 2868w, 1970vw, 1965vw, 1941vw, 1912vw, 1641w, 1598w, 1584w, 1563w, 1467m, 1434m, 1382w, 1354w, 1278w, 1206s, 1174s, 1119s, 1106m, 1093m, 1052w, 1032s, 1009s, 978w, 953w, 912w, 860w, 816m, 767m, 741m, 711w, 680s, 648m, 562s, 494w, 466w. HRMS (ESI) m/z: [M⁺] (C₁₁H₁₇INO⁺) calculated: 306.034934, found: 306.03521.

### Example 20

### Preparation of 1-(2-hydroxyethyl)-1-(4-iodobenzyl)pyrrolidin-1-ium 4-methylbenzenesulfonate (20)

Pale-yellow viscous liquid which crystallized upon cooling (453 mg, 0.90 mmol, 78 %). 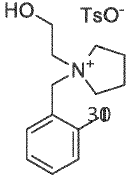 M.p. 97-99 °C. ¹H NMR (300 MHz, DMSO-*d*₆) δ 8.07 (dd, *J* = 8.0, 1.2 Hz, 1H), 7.74 (dd, *J* = 7.8, 1.6 Hz, 1H), 7.54 (ddd, *J* = 7.5, 7.5, 1.3 Hz, 1H), 7.48 (d, *J* = 7.9 Hz, 2H), 7.26 (ddd, *J* = 7.7, 7.7, 1.7 Hz, 1H), 7.11 (d, *J* = 7.8 Hz, 2H), 5.47 (t, *J* = 4.4 Hz, 1H), 4.80 (s, 2H), 3.99 ― 3.93 (m, 2H), 3.83 ― 3.63 (m, 2H), 3.54 ― 3.45 (m, 2H), 3.44 ― 3.29 (m, 2H), 2.28 (s, 3H), 2.14 ― 1.87 (m, 4H). ¹³C NMR (75 MHz, DMSO-*d*₆) δ 145.69, 140.81, 137.63, 134.80, 132.16, 131.57, 128.94, 128.07, 125.48, 105.48, 66.43, 62.17, 60.26, 55.70, 22.41, 20.80. IR (diamond-ATR): ṽ (cm⁻¹) 3356m,br, 3038vw, 3021vw, 2982vw, 2965vw, 2916vw, 2902vw, 2874vw, 2849vw, 1978vw, 1965vw, 1954vw, 1923vw, 1900vw, 1668vw, 1646vw, 1598vw, 1585vw, 1598vw, 1585vw, 1562vw, 1496w, 1470w, 1457m, 1438w, 1356w, 1306w, 1277w, 1256vw, 1214m, 1186s, 1120s, 1106m, 1075m, 1051w, 1036s, 1013s, 993m, 959m, 908w, 892w, 875w, 822m, 799w, 765m, 730w, 713w, 696w, 681s, 649w, 626w, 590w, 563s, 547m, 521w, 450w. HRMS (ESI) m/z: [M⁺] (C₁₃H₁₉INO⁺) calculated: 332.050584, found: 332.05093. Elem. anal. C₂₀H₂₆INO₄S, calculated: C (47.72), H (5.21), N (2.78), S (6.37), found: C, (47.68), H (5.21), N (2.78), S (6.31).

### Example 21

### Preparation of [3-(4-hexyloxy-3-iodophenyl)propyl]-(2-hydroxyethyl)-dimethylammonium 4-methylbenzenesulfonate (21)

The alkylating agent **I** (338 mg, 0.655 mmol, 1 eq.), DMF (0.8 mL), and 2-(dimethylamino)ethanol (350 µL, 3.48 mmol, 5.3 eq.) were used for the synthesis of compound **21**. The reaction time was 2 h at the temperature of 75 °C. Precipitation was performed from a dichloromethane solution with hexane (45 mL), the product was then re-dissolved in a mixture of dichloromethane and MeOH (2 mL, 9:1 v/v) and precipitated with a mixture of Et₂O and hexane. The product was then once more re-dissolved in pure dichloromethane and precipitated with pure hexane (45 mL). The product was obtained as a white solid (391 mg, 0.65 mmol, 99 %). 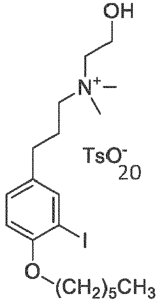 ¹H NMR (300 MHz, DMSO-*d*₆) δ 7.66 (d, *J* = 2.1 Hz, 1H), 7.49 (d, *J* = 8.0 Hz, 2H), 7.21 (dd, *J* = 8.4, 2.1 Hz, 1H), 7.11 (d, *J* = 7.9 Hz, 2H), 6.91 (d, *J* = 8.4 Hz, 1H), 5.30 (t, *J* = 5.0 Hz, 1H), 3.98 (t, *J* = 6.2 Hz, 2H), 3.81 (br s, 2H), 3.42 ― 3.28 (m, 4H), 3.06 (s, 6H), 2.49 (t, *J* = 7.4 Hz, 2H), 2.28 (s, 3H), 2.02 ― 1.88 (m, 2H), 1.77 ― 1.65 (m, 2H), 1.53 ― 1.40 (m, 2H), 1.37 ― 1.24 (m, 4H), 0.93 ― 0.84 (m, 3H), ¹³C NMR (75 MHz, DMSO-*d*₆) δ 155.62, 145.74, 138.43, 137.61, 134.57, 129.56, 128.06, 125.48, 112.43, 86.81, 68.63, 64.63, 63.63, 54.95, 50.90, 30.88, 30.25, 28.53, 25.22, 23.84, 22.08, 20.78, 13.91.

### Example 22

### Preparation of [3-(4-decyloxy-3-iodophenyl)propyl]-(2-hydroxyethyl)-dimethylammonium methanesulfonate (22)

The alkylating agent **J** (250 mg, 0.504 mmol, 1 eq.), DMF (0.7 mL), and 2-(dimethylamino)ethanol (200 µL, 1.99 mmol, 3.9 eq.) were used for the synthesis of compound **22**. The reaction was run 1 h at the temperature of 65 °C, the temperature was then for increased to 75 °C for 45 min. However, as the conversion was still not complete, the temperature was increased to 82 °C for additional 30 min. The product was purified by precipitation from a MeOH solution using a mixture of Et₂O and hexane (1:4 v/v). The product was obtained as a white waxy solid (186 mg, 0.318 mmol, 63 %). 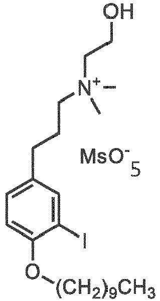 ¹H NMR (600 MHz, DMSO-*d6*) δ 7.67 (s, 1H), 7.21 (d, *J* = 8.4 Hz, 1H), 6.92 (d, *J* = 8.4 Hz, 1H), 5.28 (t, *J* = 4.9 Hz, 1H), 3.98 (t, *J* = 6.1 Hz, 2H), 3.81 (br s, 2H), 3.40 - 3.37 (m, 2H), 3.35 - 3.30 (m, 2H), 3.06 (s, 6H), 2.49 (t, *J* = 7.5 Hz, 2H), 2.30 (s, 3H), 2.00 - 1.92 (m, 2H), 1.75 - 1.67 (m, 2H), 1.49 - 1.42 (m, 2H), 1.36 - 1.20 (m, 12H), 0.86 (t, *J* = 6.6 Hz, 3H). ¹³C NMR (151 MHz, CDCl₃) δ 156.57, 139.05, 133.57, 129.55, 112.29, 87.02, 69.49, 65.78, 65.27, 56.24, 51.95, 51.94, 39.77, 32.02, 30.87, 29.69, 29.67, 29.45, 29.45, 29.22, 26.20, 24.80, 22.81, 14.26.

### Example 23

### Preparation of (2-hydroxyethyl)-[2-(2-iodophenoxy)ethyl]-dimethylammonium bromide (23)

The alkylating agent **K** (350 mg, 1.07 mmol, 1 eq.), DMF (1 mL), and 2-(dimethylamino)ethanol (646 µL, 6.42 mmol, 6.0 eq.) were used for the synthesis of compound **23**. The reaction time was 1 h 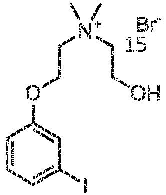 at the temperature of 67 °C. The product was obtained as a white solid (398 mg, 0.96 mmol, 90 %). ¹H NMR (600 MHz, DMSO-*d6*) δ 7.80 (dd, *J* = 7.7, 1.5 Hz, 1H), 7.42 ― 7.38 (m, 1H), 7.09 (dd, *J* = 8.2, 0.8 Hz, 1H), 6.81 (ddd, *J* = 7.7, 7.7, 1.1 Hz, 1H), 5.35 (t, *J* = 4.9 Hz, 1H), 4.55 ― 4.50 (m, 2H), 3.95 ― 3.88 (m, 4H), 3.64 ― 3.60 (m, 2H), 3.28 (s, 6H).

### Example 24

### Preparation of 1-(2-hydroxyethyl)-1-[2-(2-iodophenoxy)ethyl]pyrrolidin-1-ium bromide (24)

The alkylating agent **K** (350 mg, 1.07 mmol, 1 eq.), DMF (1 mL), and 2-(pyrrolidin-1-yl)ethanol (376 µL, 3.21 mmol, 3.0 eq.) were used for the synthesis of compound **24**. The reaction time was 90 min at the temperature of 77 °C. The product was obtained as a pale-yellow viscous liquid (331 mg, 0.75 mmol, 70 %). 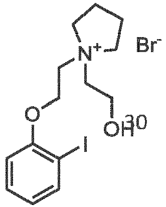 ¹H NMR (300 MHz, DMSO-*d6*) δ 7.81 (dd, *J* = 7.7, 1.6 Hz, 1H), 7.41 (ddd, *J =* 8.3, 7.4, 1.6 Hz, 1H), 7.06 (dd, *J* = 8.3, 1.2 Hz, 1H), 6.81 (ddd, *J* = 7.7, 7.4, 1.2 Hz, 1H), 5.34 (t, *J* = 4.8 Hz, 1H), 4.54 - 4.46 (m, 2H), 3.95 - 3.85 (m, 4H), 3.83 - 3.65 (m, 4H), 3.63 - 3.55 (m, 2H), 2.23 - 2.06 (m, 4H).

### Example 25

### Preparation of (2-hydroxyethyl)-[2-(4-iodophenoxy)ethyl]-dimethylammonium bromide (25)

The alkylating agent **L** (350 mg, 1.07 mmol, 1 eq.), DMF (1 mL), and 2-(dimethylamino)ethanol 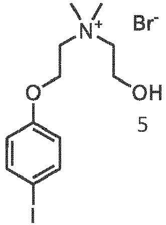 (646 µL, 6.42 mmol, 6.0 eq.) were used for the synthesis of compound **25**. The reaction time was 3 h at the temperature of 70 °C. The product was obtained as a white solid (384 mg, 0.92 mmol, 86 %). ¹H NMR (300 MHz, DMSO-*d6*) δ 7.67 - 7.61 (m, 2H), 6.90 - 6.83 (m, 2H), 5.31 (t, *J* = 4.9 Hz, 1H), 4.51 - 4.39 (m, 2H), 3.92 - 3.80 (m, 4H), 3.58 - 3.50 (m, 2H), 3.20 (s, 6H).

### Example 26

### Preparation of 1-(2-hydroxyethyl)-1-[2-(4-iodophenoxy)ethyl]pyrrolidin-1-ium bromide (26)

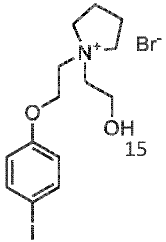

The alkylating agent **L** (350 mg, 1.07 mmol, 1 eq.), DMF (1 mL), and 2-(pyrrolidin-1-yl)ethanol (376 µL, 3.21 mmol, 3.0 eq.) were used for the synthesis of compound **26**. The reaction time was 3 h at the temperature of 70 °C. The product was obtained as a pale-yellow solid (380 mg, 0.86 mmol, 80 %). ¹H NMR (600 MHz, DMSO-*d6*) δ 7.64 (d, *J* = 8.8 Hz, 2H), 6.86 (d, *J* = 8.8 Hz, 2H), 5.33 (t, *J* = 4.9 Hz, 1H), 4.43 (t, *J* = 4.5 Hz, 2H), 3.88 - 3.83 (m, 4H), 3.74 - 3.67 (m, 2H), 3.67 - 3.61 (m, 2H), 3.53 - 3.49 (m, 2H), 2.12 - 2.05 (m, 4H).

### Example 27

### Preparation of (2-hydroxyethyl)-[2-(3-iodophenoxy)ethyl]-dimethylammonium bromide (27)

The alkylating agent **M** (300 mg, 0.918 mmol, 1 eq.), DMF (1 mL), and 2-(dimethylamino)ethanol 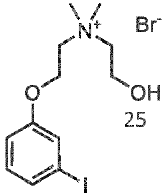 (554 µL, 5.51 mmol, 6.0 eq.) were used for the synthesis of compound **27**. The reaction time was 2 h at the temperature of 70 °C. The product was obtained as a white solid (368 mg, 0.88 mmol, 96 %). ¹H NMR (300 MHz, DMSO-*d6*) δ 7.40 ― 7.34 (m, 2H), 7.15 ― 7.08 (m, 1H), 7.05 ― 7.00 (m, 1H), 5.32 (t, *J* = 5.0 Hz, 1H), 4.51 ― 4.42 (m, 2H), 3.92 ― 3.81 (m, 4H), 3.58 ― 3.51 (m, 2H), 3.20 (s, 6H).

### Example 28

### Preparation of 1-(2-hydroxyethyl)-1-[2-(3-iodophenoxy)ethyl]pyrrolidin-1-ium bromide (28)

The alkylating agent **M** (300 mg, 0.918 mmol, 1 eq.), DMF (1 mL), and 2-(pyrrolidin-1-yl)ethanol (322 µL, 2.75 mmol, 3.0 eq.) were used for the synthesis of compound **28**. The reaction time was 2 h at the temperature of 70 °C. The product was obtained as a yellow viscous liquid which crystallized upon cooling (380 mg, 0.86 mmol, 80 %). 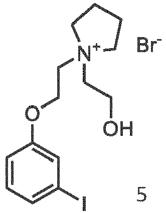 ¹H NMR (300 MHz, DMSO-*d6*) δ 7.40 ― 7.33 (m, 2H), 7.15 ― 7.07 (m, 1H), 7.05 ― 6.98 (m, 1H), 5.32 (t, *J* = 5.0 Hz, 1H), 4.45 (t, *J* = 4.7 Hz, 2H), 3.92 ― 3.79 (m, 4H), 3.78 ― 3.58 (m, 4H), 3.56 ― 3.47 (m, 2H), 2.17 ― 2.02 (m, 4H).

### Example 29

### Preparation of (2-hydroxyethyl)-[6-(3-iodophenoxy)hexyl]-dimethylammonium bromide (29)

The alkylating agent **N** (350 mg, 0.914 mmol, 1 eq.), DMF (1 mL), and 2-(dimethylamino)ethanol 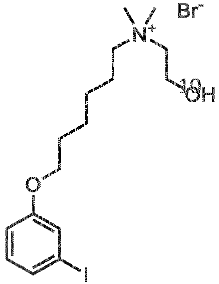 (552 µL, 5.48 mmol, 6.0 eq.) were used for the synthesis of compound **29**. The reaction time was 2 h at the temperature of 70 °C. The product was obtained as a white solid (395 mg, 0.84 mmol, 92 %). ¹H NMR (300 MHz, DMSO-*d6*) δ 7.30 - 7.24 (m, 2H), 7.10 - 7.03 (m, 1H), 6.98 - 6.91 (m, 1H), 5.27 (t, *J* = 5.0 Hz, 1H), 3.96 (t, *J* = 6.4 Hz, 2H), 3.87 - 3.76 (m, 2H), 3.46 - 3.28 (m, 4H), 3.07 (s, 6H), 1.78 - 1.62 (m, 4H), 1.51 - 1.39 (m, 2H), 1.39 - 1.24 (m, 2H).

### Example 30

### Preparation of 1-(2-hydroxyethyl)-1-[6-(3-iodophenoxy)hexyl]pyrrolidin-1-ium bromide (30)

The alkylating agent **N** (350 mg, 0.914 mmol, 1 eq.), DMF (1 mL), and 2-(pyrrolidin-1-yl)ethanol 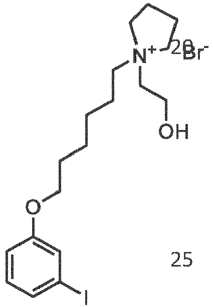 (320 µL, 2.74 mmol, 3.0 eq.) were used for the synthesis of compound **30**. The reaction time was 2 h at the temperature of 70 °C. The product was obtained as a pale-yellow solid (399 mg, 0.88 mmol, 80 %). ¹H NMR (300 MHz, DMSO-*d6*) δ 7.30 ― 7.24 (m, 2H), 7.10 ― 7.02 (m, 1H), 6.98 ― 6.91 (m, 1H), 5.28 (t, *J* = 5.1 Hz, 1H), 3.96 (t, *J* = 6.4 Hz, 2H), 3.85 ― 3.73 (m, 2H), 3.65 ― 3.44 (m, 4H), 3.42 ― 3.26 (m, 4H), 2.11 ― 1.97 (m, 4H), 1.78 ― 1.62 (m, 4H), 1.51 ― 1.39 (m, 2H), 1.39 ― 1.25 (m, 2H).

### Example 31

### Preparation of (2-hydroxyethyl)-[4-(2-iodophenoxy)butyl]-dimethylammonium bromide (31)

The alkylating agent **O** (355 mg, 1.00 mmol, 1 eq.), DMF (1 mL), and 2-(dimethylamino)ethanol (604 µL, 6.00 mmol, 6.0 eq.) were used for the synthesis of compound **31**. The reaction time was 2.5 h at the temperature of 70 °C. The product was obtained as a colorless viscous liquid (418 mg, 0.94 mmol, 94 %). 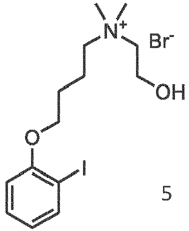 ¹H NMR (300 MHz, DMSO-*d6*) δ 7.77 (dd, *J* = 7.7, 1.6 Hz, 1H), 7.36 (ddd, *J* = 8.2, 7.4, 1.6 Hz, 1H), 7.01 (dd, *J* = 8.3, 1.3 Hz, 1H), 6.79 ― 6.72 (m, 1H), 5.28 (t, *J* = 5.0 Hz, 1H) 4.08 (t, *J* = 6.4 Hz, 2H), 3.90 ― 3.79 (m, 2H), 3.52 ― 3.37 (m, 4H), 3.10 (s, 6H), 2.01 ― 1.86 (m, 2H), 1.86 ― 1.71 (m, 2H).

### Example 32

### Preparation of 1-(2-hydroxyethyl)-1-[4-(2-iodophenoxy)butyl]pyrrolidin-1-ium bromide (32)

The alkylating agent **O** (355 mg, 1.00 mmol, 1 eq.), DMF (1 mL), and 2-(pyrrolidin-1-yl)ethanol (468 µL, 4.00 mmol, 4.0 eq.) were used for the synthesis of compound **32**. The reaction time was 2.5 h 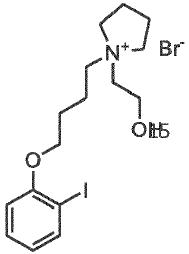 at the temperature of 70 °C. The product was obtained as a yellow viscous liquid (388 mg, 0.83 mmol, 83 %). ¹H NMR (300 MHz, DMSO-*d6*) δ 7.76 (dd, *J* = 7.7, 1.6 Hz, 1H), 7.35 (ddd, *J =* 8.3, 7.4, 1.6 Hz, 1H), 7.01 (dd, *J* = 8.3, 1.3 Hz, 1H), 6.78 - 6.72 (m, 1H), 5.28 (t, *J* = 5.1 Hz, 1H), 4.07 (t, *J* = 5.9 Hz, 2H), 3.86 - 3.77 (m, 2H), 3.72 - 3.59 (m, 2H), 3.59 - 3.38 (m, 6H), 2.14 - 2.00 (m, 4H), 1.99 - 1.85 (m, 2H) 1.85 - 1.73 (m, 2H).

### Example 33

### Preparation of (2-hydroxyethyl)-[2-(5-iodothiophen-2-yl)ethyl]-dimethylammonium 4-methylbenzenesulfonate (33)

The alkylating agent **P** (250 mg, 0.612 mmol, 1 eq.), DMF (1 mL), and 2-(dimethylamino)ethanol (370 µL, 3.67 mmol, 6.0 eq.) were used for the synthesis of compound **33**. The reaction time was 2 h at the temperature of 65 °C. The product was obtained as a white solid (273 mg, 0.55 mmol, 90 %). ¹H NMR (300 MHz, DMSO-*d6*) δ 7.48 (d, *J =* 8.0 Hz, 2H), 7.22 (d, *J* = 3.6 Hz, 1H), 7.11 (d, *J =* 8.0 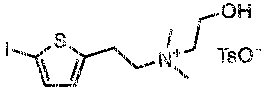 Hz, 2H), 6.74 (d, *J* = 3.6 Hz, 1H), 5.33 (t, *J* = 4.9 Hz, 1H), 3.89 - 3.80 (m, 2H), 3.63 - 3.53 (m, 2H), 3.50 - 3.43 (m, 2H), 3.35 - 3.26 (m, 2H), 3.12 (s, 3H), 2.29 (s, 3H).

### Example 34

### Preparation of 1-(2-hydroxyethyl)-1-[2-(5-iodothiophen-2-yl)ethyl]pyrrolidin-1-ium 4-methylbenzenesulfonate (34)

The alkylating agent P (250 mg, 0.612 mmol, 1 eq.), DMF (1 mL), and 2-(pyrrolidin-1-yl)ethanol (358 µL, 3.06 mmol, 5.0 eq.) were used for the synthesis of compound 34. The reaction time was 2 h at the temperature of 65 °C. The product was obtained as brown solid (120 mg, 0.23 mmol, 37 %). 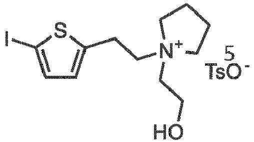 ¹H NMR (300 MHz, DMSO) δ 7.47 (d, *J* = 8.0 Hz, 2H), 7.22 (d, *J* = 3.6 Hz, 1H), 7.11 (d, *J* = 8.0 Hz, 1H), 6.75 (d, *J* = 3.6 Hz, 1H), 5.36 (t, *J* = 4.8 Hz, 2H), 3.90 ― 3.76 (m, 2H), 3.70 ― 3.51 (m, 6H), 3.51 ― 3.43 (m, 2H), 2.29 (s, 3H), 2.13 ― 2.00 (m, 4H).

### Example 35

### Preparation of 4-(2-hydroxyethyl)-4-(4-iodobenzyl)morpholin-4-ium bromide (35)

The alkylating agent **F** (300 mg, 1.01 mmol, 1 eq.) and 4-(2-hydroxyethyl)morpholine (4.00 mL, 33,0 mmol, 32.7 eq.) were used for the synthesis of compound **35**. The reaction time was 18 h at the 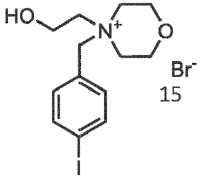 temperature of 72 °C. The product was obtained as an amorphous pink solid (418 mg, 0.98 mmol, 97 %). ¹H NMR (300 MHz, DMSO-*d6*) δ 7.89 (d, *J* = 8.3 Hz, 2H), 7.39 (d, *J* = 8.3 Hz, 2H), 5.49 (t, *J* = 4.7 Hz, 1H), 4.81 (s, 2H), 4.03 ― 3.90 (m, *J* = 3.6 Hz, 6H), 3.54 ― 3.40 (m, 6H).

### Example 36

### Preparation of 4-(2-hydroxyethyl)-4-(2-iodobenzyl)morpholin-4-ium chloride (36)

The alkylating agent **G** (300 mg, 1.19 mmol, 1 eq.) and 4-(2-hydroxyethyl)morpholine (4.00 mL, 33,0 mmol, 27.7 eq.) were used for the synthesis of compound **36**. The reaction time was 18 h at the temperature of 72 °C. The product was obtained as an amorphous brown solid (364 mg, 0.948 mmol, 80 %). 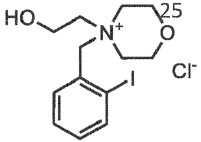 ¹H NMR (300 MHz, DMSO-*d6*) δ 8.12 - 8.06 (m, 1H), 7.81 - 7.74 (m, 1H), 7.61 - 7.51 (m, 1H), 7.32 - 7.24 (m, 1H), 5.86 (t, *J =* 5.0 Hz, 1H), 4.96 (s, 2H), 4.14 - 3.96 (m, 4H), 3.95 - 3.82 (m, 4H), 3.77 - 3.68 (m, 2H), 3.41 - 3.27 (m, 2H).

### Example 37

### Preparation of (2-hydroxyethyl)-{6-[(2-iodopyridin-3-yl)oxy]hexyl}-dimethylammonium bromide (37) - not part of the invention

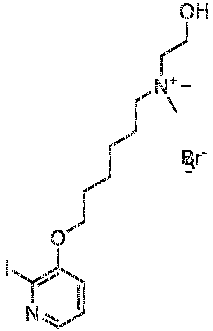

The alkylating agent **Q** (200 mg, 0.521 mmol, 1 eq.) and 2-(dimethylamino)ethanol (2.00 mL, 19.9 mmol, 38.2 eq.) were used for the synthesis of compound **37**. The reaction time was 40 min at the temperature of 75 °C. The product was obtained as a colorless viscous liquid (240 mg, 0.51 mmol, 97 %). ¹H NMR (300 MHz, DMSO-*d6*) δ 7.95 (dd, *J* = 3.0, 3.0 Hz, 1H), 7.37 ― 7.32 (m, 2H), 5.27 (t, *J* = 4.9 Hz, 1H), 4.08 (t, *J* = 6.2 Hz, 2H), 3.87 ― 3.77 (m, 2H), 3.44 ― 3.29 (m, 4H), 3.08 (s, 6H), 1.83 ― 1.65 (m, 4H), 1.59 ― 1.46 (m, 2H), 1.42 ― 1.28 (m, 2H).

### Example 38

### Preparation of (2-hydroxyethyl)-{4-[(2-iodo-6-methylpyridin-3-yl)oxy]butyt}-dimethylammonium bromide (38) ― not part of the invention

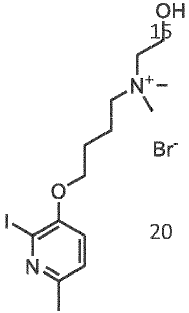

The alkylating agent **R** (200 mg, 0.540 mmol, 1 eq.) and 2-(dimethylamino)ethanol (2.00 mL, 19.9 mmol, 36.9 eq.) were used for the synthesis of compound **38**. The reaction time was 40 min at the temperature of 75 °C. The product was obtained as a colorless viscous liquid (246 mg, 0.54 mmol, 99 %). ¹H NMR (300 MHz, DMSO-*d6*) δ 7.26 (d, *J* = 8.2 Hz, 1H), 7.19 (d, *J* = 8.2 Hz, 1H), 5.29 (t, *J* = 4.9 Hz, 1H), 4.08 (t, *J* = 6.3 Hz, 2H), 3.90 - 3.76 (m, 2H), 3.52 - 3.32 (m, 4H), 3.10 (s, 6H), 2.36 (s, 3H), 1.97 - 1.84 (m, 2H), 1.82 - 1.69 (m, 2H).

### Example 39

### Preparation of (2-hydroxyethyl)-{4-[(3-iodo-[1,1'-biphenyl]-4-yl)oxy]butyl}-dimethylammonium bromide (39) - not part of the invention

The alkylating agent **S** (200 mg, 0.464 mmol, 1 eq.) and 2-(dimethylamino)ethanol (2.00 mL, 19.9 mmol, 42.9 eq.) were used for the synthesis of compound **39**. The reaction time was 40 min at the temperature of 75 °C. The product was obtained as a colorless viscous liquid which crystallized upon 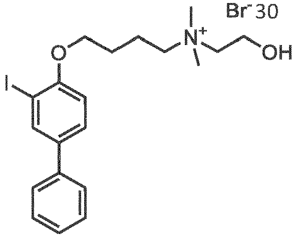 cooling (238 mg, 0.46 mmol, 99 %). ¹H NMR (300 MHz, DMSO-*d6*) δ 8.04 (d, *J* = 2.3 Hz, 1H), 7.66 (dd, *J =* 8.5, 2.3 Hz, 1H), 7.64 - 7.57 (m, *J* = 7.3, 1.3 Hz, 2H), 7.47 - 7.39 (m, 2H), 7.36 - 7.30 (m, 1H), 7.09 (d, *J* = 8.5 Hz, 1H), 5.30 (t, *J* = 5.0 Hz, 1H), 4.13 (t, *J* = 5.9 Hz, 2H), 3.90 - 3.81 (m, 2H), 3.55 - 3.39 (m, 4H), 3.12 (s, 6H), 2.03 - 1.88 (m, 2H), 1.87 - 1.70 (m, 2H).

### Example 40

### Preparation of (2-hydroxyethyl)-[6-(4-iodo-3-nitrophenoxy)hexyl]-dimethylammonium bromide (40)

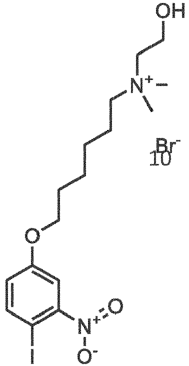

The alkylating agent **T** (300 mg, 0.700 mmol, 1 eq.) and 2-(dimethylamino)ethanol (2.00 mL, 19.9 mmol, 28.4 eq.) were used for the synthesis of compound 4. The reaction time was 40 min at the temperature of 75 °C. The product was obtained as a bright yellow solid (362 mg, 0.70 mmol, 100 %). ¹H NMR (300 MHz, DMSO-*d6*) δ 7.92 (d, *J* = 8.7 Hz, 1H), 7.52 (d, *J* = 2.9 Hz, 1H), 7.04 (dd, *J* = 8.7, 2.9 Hz, 1H), 5.27 (t, *J* = 5.0 Hz, 1H), 4.04 (t, *J* = 6.4 Hz, 2H), 3.87 ― 3.76 (m, 2H), 3.46 ― 3.29 (m, 4H), 3.08 (s, 6H), 1.80 ― 1.63 (m, 4H), 1.51 ― 1.38 (m, 2H), 1.38 ― 1.24 (m, 2H).

### II. Synthesis of radiolabeled substances

### Example 23

Solution A: PdCl₂ (1 mg) and acetonitrile (1 mL) were put into 2 mL micro-centrifugation tube, the resulting suspension was agitated in an ultrasonic bath for 15 min. The obtained suspension was then centrifuged and decanted from the non-dissolved solid PdCl₂.
Solution B: Ascorbic acid (30 mg) in deionized water (1 mL)
Solution C: Compound **4** (1 mg) in deionized water (100 µL)

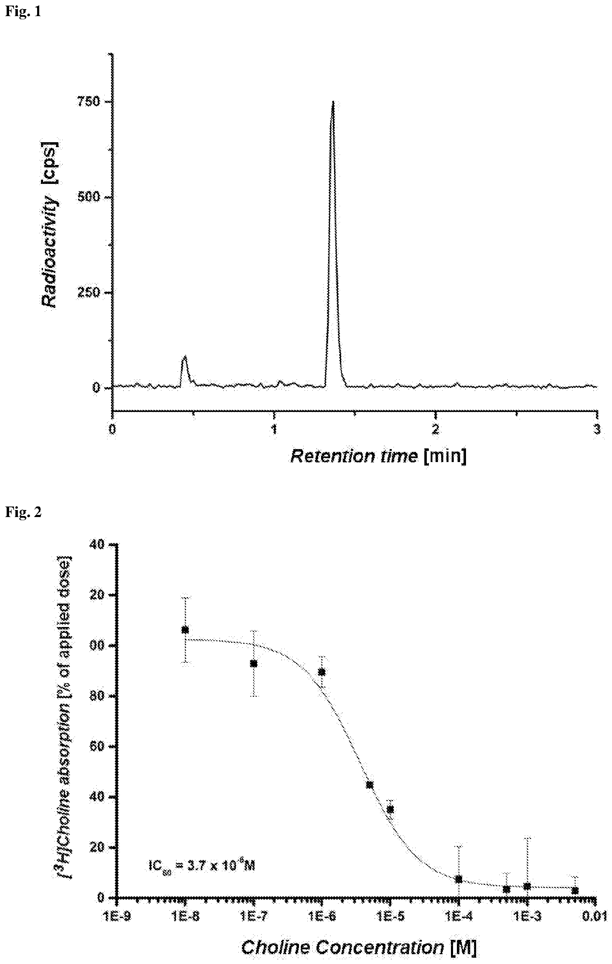

The solutions A (4 µL), B (2 µL), and C (1 µL) were put into a 0.5 mL microcentrifuge tube. The tube was gently shaken, the mixture was left for 15 min at the room temperature and was subsequently centrifuged. Then, an aqueous solution of Na¹²⁵I (8 µL, 74.0 MBq) was added, and the reaction mixture was gently shaken. The tube was sealed by a plastic film, centrifuged, and subsequently it was immersed into a water bath preheated to 75 °C. After 1 h, the tube was removed from the bath, centrifuged, and immersed into the bath again so that the solvent vaporized at the top of the tube was returned back into the remaining solution. After another 1 h, the tube was removed, cooled to room temperature, and the reaction mixture was diluted with deionized water (100 µL). Strong anion exchange resin in the TsO⁻ cycle (5 grains, particle size 650 - 820 µm, 1 meq. mL⁻¹) was inserted to the tube to remove most of the free ¹²⁵I⁻. The grains of anion exchange resin were subsequently decanted, the solution was transferred into a clean centrifugation tube, and the grains of the anion exchange resin were rinsed with deionized water (2 × 50 µL), which was then added to the first solution. The resulting solution was filtered through a 0.20 µm syringe filter. The radio-labeled substance **4** (55.8 MBq, 75 %) was obtained in a good radiochemical purity according to the radio-HPLC analysis (Figure 1).

### III. Biological tests

### In vitro determination of IC₅₀ values of the choline analogs

IC₅₀ value corresponds to a concentration of the given analog of choline which reduces the accumulation of choline chloride, the natural ligand of choline transporterss, to 50 % of the maximum value. For the assay, the tritium labeled choline chloride, i.e. [³H]choline chloride, was used. The assay was performed *in vitro* on the cell line PC-3 derived from a human prostate carcinoma, which is known to overexpress choline transporters. For the determination of IC₅₀, the PC-3 cells were cultivated in 24-well cultivation plates to confluence of 90 %. As the negative control, a cell line of human ovarian carcinoma SKOV3 prepared to the confluence of 90 % in 24-well plates was used.

The cultivation medium was removed from the thus prepared cells in cultivation plates and the cells were rinsed with phosphate buffered saline (PBS). Subsequently, 0.3 mL of "incubation buffer" (25 mM Tris/HCl, 5.4 mM KCl, 1.8 mM CaCl₂, 0.8 mM MgSO₄, 5 mM glucose, 140 mM NaCl, pH adjusted to 7.4) was added to the cells. Then 0.1 mL of the solution of the tested substance (analog of choline) in the incubation buffer was added to the PC-3 cells in such concentrations, that after adding [³H]choline chloride the resulting concentrations of the tested analog would be following: 0 M, 10⁻⁸ M, 10⁻⁷ M, 10⁻⁶ M, 5 × 10⁻⁶ M, 10⁻⁵ M, 10⁻⁴ M, 5 × 10⁻⁴ M, 10⁻³ M, 5×10⁻³ M. Each concentration was tested in triplicate.

To the SKOV3 cells, 0.1 mL of the tested compound (analog of choline) solution in the incubation buffer was added in such concentrations, that after the addition of [³H]choline chloride the concentrations of the analog of choline in the medium with cells were 0 M and 10⁻⁴ M. Each concentration was tested in triplicate.

Subsequently, all cells prepared in this manner (PC-3 and SKOV3) were incubated with the analogs of choline for 15 minutes at the temperature of 37 °C.

Then 0.1 mL of the solution of the [³H]choline chloride in the incubation buffer was added to all cell-containing wells, so that 0.1 mL of the solution had the activity of 15 kBq and that the final concentration of the [³H]choline chloride in the medium with the cells was 0.81 × 10⁻⁶ M. The cells were then incubated at 37 °C for a period of 60 min. After the incubation, all buffer solution was removed from the cells. The cells were rinsed with PBS, which was subsequently removed, and 0.2 mL of aqueous NaOH solution (0.1 M) was added to each well. The cells were incubated with sodium hydroxide for 30 min at 37 °C. The lysate was then homogenized with a pipette and 10 µL of the lysate were used for the determination of protein concentration by bicinchonic acid assay (BCA).

The remaining lysate (180 µL) was quantitatively transferred into scintillation vials containing a previously prepared scintillation solution (2 mL of the solution in each vial). Each well was then additionally rinsed with 0.2 mL of pure water and this solution was added to the corresponding scintillation vials. Scintillation vials were closed and their content was stirred on a shaker.

To another three scintillation vials containing scintillation solution (2 mL of the solution in each vial) the standard samples of [³H]choline chloride were added (i.e. 10 µ1 of the [³H]choline chloride solution together with 180 µl of aqueous NaOH solution (0.1 M) and 200 µl of water). The vials were closed and their content was stirred on the shaker.

Subsequently, radioactivity of each scintillation vial was measured using a liquid scintillation counter (Tri-Carb 2910TR Liquid Scintillation Counter, PerkinElmer). The recorded counts per second (cps) were then used to calculate the accumulation of [³H]choline chloride in the individual cell samples. These results were corrected to the applied amount of radioactivity ([³H]choline chloride standards) and the quantity of proteins in each cell samples. These values were used to construct a sigmoid curve of dependence between the rate of [³H]choline chloride accumulation and the competing ligand (analog of choline) concentration(inhibition curve, Figure 2). The IC₅₀ values were calculated from these inhibition curves.

### Assessment of the IC₅₀ values obtained in vitro for the analogs of choline

Using the above-described method, the IC₅₀ values were determined for 40 analogs of choline according to the present invention, and also for hemicholinium-3, fluoromethylcholine, and choline (refer to Table 1). Choline was chosen as it is a natural ligand of choline transporters, hemicholinium-3 was chosen as a classic representative of the inhibitors of choline transporters, and the radioactive fluorine (¹⁸F) labeled fluoromethylcholine is routinely used in nuclear medicine to diagnose prostate carcinoma. The IC₅₀ values determined for hemicholinium-3 and choline were in accordance with the published data (7.3 × 10⁻⁶ mol . L-¹ for choline and 1.7 × 10⁻⁵ mol . L-¹ for hemicholinium-3 [Müller S. A. et al. (2009), Eur. J. Nucl. Med. Mol. Imaging 36, 1434-1442]). Twenty-six derivatives of choline according to the the present invention possessed the same or higher IC₅₀ values than hemicholinium-3, and eighteen derivatives of choline according to the present invention exhibited higherIC₅₀ values than fluoromethylcholine, which is routinely used in nuclear medicine to diagnose prostate carcinoma. In the case of eight derivatives according to the present invention, the IC₅₀ values were even higher than the IC₅₀ value determined for choline (natural ligand).

### Determination of basic pharmacological properties of analogs of choline

For twenty of the tested analogs of choline, stability in the plasma, binding to plasmatic proteins, microsomal stability and the permeability through artificial membrane (PAMPA) (Table 2) were determined. Most of the analogs of choline under study have demonstrated sufficient stability in plasma after two-hour incubation (> 70 to 80 % of the original quantity of the substance). The binding to plasmatic proteins influences mainly the distribution of the drug in the organism. Plasma protein binding has shown relatively higher values (> 20 % bound to plasmatic proteins) for six analogs of choline under study. More than a half of the tested derivatives of choline was quite quickly (internal clearance > 50 µL . min⁻¹ . mg⁻¹) metabolized by hepatic microsomes. All studied compounds under exhibited a low ability to pass across the artificial membrane by passive diffusion. This is a desirable property, which enables a specific distribution of these substances and further supports their planned diagnostic or therapeutic use (choline transporters are localized on the plasmatic membrane of the cells). In general, the substances have shown suitable pharmacological properties for both diagnostic and therapeutic applications.

**Table 1. IC₅₀ of the novel derivatives**

| **Choline analog** | **IC₅₀ (mol. L⁻¹)** |
|---|---|
| Example 1 | 4.7×10⁻⁵ |
| Example 2 | 9.2×10⁻⁵ |
| Example 3 | 2.5×10⁻⁵ |
| Example 4 | 1.9×10⁻⁵ |
| Example 5 | 7.4×10⁻⁶ |
| Example 6 | 8.3×10⁻⁵ |
| Example 7 | 1.6×10⁻⁵ |
| Example 8 | 1.2×10⁻⁴ |
| Example 9 | 9.9×10⁻⁵ |
| Example 10 | 1.3×10⁻⁵ |
| Example 11 | 1.5×10⁻⁴ |
| Example 12 | 8.0×10⁻⁵ |
| Example 13 | 2.9×10⁻⁵ |
| Example 14 | 1.9×10⁻⁴ |
| Example 15 | 8.4×10⁻⁶ |
| Example 16 | 2.4×10⁻⁴ |
| Example 17 | 7.4×10⁻⁴ |
| Example 18 | 1.8×10⁻⁴ |
| Example 19 | 1.6×10⁻⁴ |
| Example 20 | 6.3×10⁻⁶ |
| Example 21 | 4.3×10⁻⁷ |
| Example 22 | 1.9×10⁻⁶ |
| Example 23 | 2.5-10⁻⁵ |
| Example 24 | 4.7×10⁻⁶ |
| Example 25 | 2.3×10⁻⁵ |
| Example 26 | 1.8×10⁻⁵ |
| Example 27 | 2.2×10⁻⁵ |
| Example 28 | 1.5×10⁻⁵ |
| Example 29 | 4.4×10⁻⁸ |
| Example 30 | 9.1×10⁻⁷ |
| Example 31 | 1.1×10⁻⁵ |
| Example 32 | 5.1×10⁻⁶ |
| Example 33 | 4.5×10⁻⁷ |
| Example 34 | 1.0×10⁻⁵ |
| Example 35 | 2.7×10⁻⁴ |
| Example 36 | 4.3×10⁻⁵ |
| Example 37 | 1.4×10⁻⁶ |
| Example 38 | 1.8×10⁻⁵ |
| Example 39 | 1.3×10⁻⁶ |
| Example 40 | 1.4×10⁻⁷ |
| Hemicholinium-3 | 2.9×10⁻⁵ |
| Fluoromethylcholine | 1.6×10⁻⁵ |
| Choline | 3.7×10⁻⁶ |

**Table 2. In vitro pharmacokinetic properties of derivatives 1-8 and 14-20**

| **Choline analog of Example** | **Plasma stability (120 min)** | **Plasmatic protein binding** | **Microsomal stability** | | **"Parallel Artificial Membrane Permeability Assay" (PAMPA)** |
|---|---|---|---|---|---|
| | **[%]** | **[%]** | ***t₁ₗ₂* [min]** | **Internal clearance [µL . min-¹ . mg⁻¹]** | ***log Pe*** |
| 1 | 28.4 | 19.5 | 165.0 | 8.4 | -9.88 |
| 2 | 59.0 | 25.2 | 97.6 | 14.2 | -9.98 |
| 3 | 76.4 | 48.8 | 1.3 | 1035.8 | -10.20 |
| 4 | 63.9 | 28.5 | 1.2 | 1123.6 | -10.50 |
| 5 | 43.0 | 34.7 | 15.6 | 88.6 | -10.26 |
| 6 | 81.3 | 26.5 | 1.1 | 1232.0 | -8.48 |
| 7 | 97.6 | 16.7 | 15.3 | 90.6 | -8.49 |
| 8 | 89.9 | 28.3 | 34.8 | 39.8 | -9.04 |
| 14 | 80.7 | 16.9 | 61.3 | 22.6 | -9.49 |
| 15 | 84.9 | 18.6 | 18.8 | 73.6 | -7.74 |
| 16 | 79.0 | 4.4 | 119.5 | 11.6 | -8.30 |
| 17 | 58.4 | 5.7 | 1732.5 | 0.8 | -7.75 |
| 18 | 73.4 | 9.7 | 22.0 | 63.0 | -7.55 |
| 19 | 91.3 | 33.9 | 8.3 | 166.4 | -10.12 |
| 20 | 81.3 | 7.88 | 4.5 | 310 | -8.44 |

### Industrial Applicability

The invention can be used in medicine for localization and treatment of several types of tumorous diseases and other disorders characterized by pathological function or expression of choline transporters.

## Claims

1. Iodinated aromatic choline analogs of general formula I 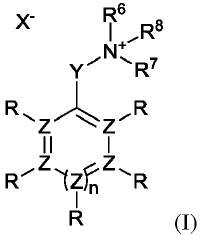 wherein
- the ring containing Z atoms is selected from benzene ring and thiophene ring;
- R are independently selected from the group consisting of H; ¹²³I; ¹²⁴I; ¹²⁵I; ¹²⁷I; ¹³¹I; C₁ to C₁₄ alkyl; C₂ to C₁₄ alkenyl; C₂ to C₁₄ alkynyl; C₆ to C₁₀ aryl; C₃ to C₆ heteroaryl comprising at least one heteroatom selected from O, S, N; C₃ to C₆ heterocyclyl comprising one to two heteroatoms selected from O, S, N; C₁ to C₁₄ alkoxy; C₆ to C₁₀ aryloxy; C₃ to C₆ heteroaryloxy comprising at least one heteroatom selected from O, S, N; C₃ to C₆ heterocyclyloxy comprising one to two heteroatoms selected from O, S, N; benzyloxy; C₁ to C₁₄ alkylthio; halogen; OH; SH; NH₂; C₁ to C₁₄ alkylamino; C₆ to C₁₀ arylamino; C₁ to C₁₄ acylamino; di(C₁ to C₁₄ alkyl)amino, wherein the alkyls are the same or different; di(C₆ to C₁₀ aryl)amino, wherein the aryls are the same or different; di(C₁ to C₁₄ acyl)amino, wherein the acyls are the same or different; CN; nitro and COORₙ, wherein Rₙ is H or C₁ to C₁₄ alkyl or aryl;
- whereas at least one of the substituents R is ¹²³I; ¹²⁴I; ¹²⁵I or ¹³¹I,
- R⁶ is 2-hydroxyethyl, 3-hydroxypropyl, or 4-hydroxybutyl;
- R⁷and R⁸ are independently selected from the group consisting of C₁ to C₆ alkyl; C₂ to C₆ alkenyl; C₂ to C₆ alkynyl; C₃ to C₈ cycloalkyl; C₃ to C₈ cycloalkenyl; C₆ to C₁₀ aryl; C₃ to C₆ heteroaryl comprising at least one heteroatom selected from O, S, N; C₃ to C₆ heterocyclyl comprising one or two heteroatoms selected from O, S, N; whereas R⁷ and R⁸ can optionally be substituted by OH group or C₁-C₄ hydroxy alkyl group; or
- R⁷and R⁸ together with the nitrogen atom form a four- to seven-membered ring, optionally comprising at least one further heteroatom selected from O, S, N, a optionally substituted by OH group or C₁-C₄ hydroxy alkyl group;
- whereas at least one, preferably exactly one, of substituents R⁶, R⁷ and R⁸ is substituted by OH group or by C₁-C₄ hydroxy alkyl group;
- Y is a linker formed by a linear or branched hydrocarbon chain having 1 to 10 carbon atoms, whereas optionally 1 to 2 carbon atoms can be replaced by oxygen and/or sulfur atoms; and
- X⁻ is a pharmaceutically acceptable anion;
with the proviso that:
- when exactly one of the substituents R is ¹²³I; ¹²⁴I; ¹²⁵I or ¹³¹I, and is in the *ortho* position, and all other substituents R are hydrogen atoms, Y is ―CH₂-, R⁶ is 2-hydroxyethyl, then NR⁷R⁸ is not dimethylamino or pyrrolidino group,
- when exactly one of the substituents R is ¹²³I; ¹²⁴I; ¹²⁵I or ¹³¹I and is in the *ortho* position, and all other substituents R are hydrogen atoms, Y is ―CH₂-, R⁶ is 2-hydroxyethyl, R⁷ is ethyl or 2-hydroxyethyl, then R⁸ is not methyl.

2. Compounds according to claim 1, wherein Y is a linear or branched hydrocarbon chain having 1 to 10 carbon atoms, whereas 1 carbon atom is replaced by oxygen; or wherein Y is linear hydrocarbon chain containing 1 to 3 carbon atoms.

3. Compounds according to any one of the preceding claims, wherein substituents R are independently selected from the group consisting of H; ¹²³I; ¹²⁴I; ¹²⁵I; ¹²⁷I; ¹³¹I; C₆ to C₁₀ aryl; C₁ to C₁₄ alkoxy; benzyloxy; NO₂.

4. Iodinated aromatic choline analogs of general formula Ia 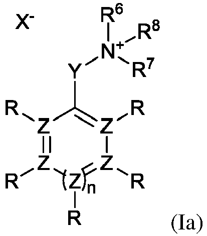 wherein
- the ring containing Z atoms is selected from benzene ring and thiophene ring;
- R are independently selected from the group consisting of H; ¹²⁷I; C₁ to C₁₄ alkyl; C₂ to C₁₄ alkenyl; C₂ to C₁₄ alkynyl; C₆ to C₁₀ aryl; C₃ to C₆ heteroaryl comprising at least one heteroatom selected from O, S, N; C₃ to C₆ heterocyclyl comprising one to two heteroatoms selected from O, S, N; C₁ to C₁₄ alkoxy; C₆ to C₁₀ aryloxy; C₃ to C₆ heteroaryloxy comprising at least one heteroatom selected from O, S, N; C₃ to C₆ heterocyclyloxy comprising one to two heteroatoms selected from O, S, N; benzyloxy; C₁ to C₁₄ alkylthio; halogen; OH; SH; NH₂; C₁ to C₁₄ alkylamino; C₆ to C₁₀ arylamino; C₁ to C₁₄ acylamino; di(C₁ to C₁₄ alkyl)amino, wherein the alkyls are the same or different; di(C₆ to C₁₀ aryl)amino, wherein the aryls are the same or different; di(C₁ to C₁₄ acyl)amino, wherein the acyls are the same or different; CN; nitro and COORₙ, wherein Rₙ is H or C₁ to C₁₄ alkyl or aryl;
- whereas at least one of the substituents R is ¹²⁷I;
- R⁶ is 2-hydroxyethyl, 3-hydroxypropyl, or 4-hydroxybutyl;
- R⁷and R⁸ are independently selected from the group consisting of C₁ to C₆ alkyl; C₂ to C₆ alkenyl; C₂ to C₆ alkynyl; C₃ to C₈ cycloalkyl; C₃ to C₈ cycloalkenyl; C₆ to C₁₀ aryl; C₃ to C₆ heteroaryl comprising at least one heteroatom selected from O, S, N; C₃ to C₆ heterocyclyl comprising one or two heteroatoms selected from O, S, N; whereas R⁷ and R⁸ can optionally be substituted by OH group or C₁-C₄ hydroxy alkyl group; or
- R⁷and R⁸ together with the nitrogen atom form a four- to seven-membered ring, optionally comprising at least one further heteroatom selected from O, S, N, a optionally substituted by OH group or C₁-C₄ hydroxy alkyl group; or
- whereas at least one, preferably exactly one, of substituents R⁶, R⁷ and R⁸ is substituted by OH group or by C₁-C₄ hydroxy alkyl group;
- Y is a linker formed by a linear or branched hydrocarbon chain having 1 to 10 carbon atoms, whereas optionally 1 carbon atom can be replaced by oxygen or sulfur atom; and
- X⁻ is a pharmaceutically acceptable anion;
with the proviso that:
- when exactly one of the substituents R is ¹²⁷I, and is in the *ortho* position, and all other substituents R are hydrogen atoms, Y is ―CH₂-, R⁶ is 2-hydroxyethyl, then NR⁷R⁸ is not dimethylamino or pyrrolidino group,
- when exactly one of the substituents R is ¹²⁷I and is in the *ortho* position, and all other substituents R are hydrogen atoms, Y is ―CH₂-, R⁶ is 2-hydroxyethyl, R⁷ is ethyl or 2-hydroxyethyl, then R⁸ is not methyl.

5. Compounds according to claim 4, wherein Y is a linear or branched hydrocarbon chain having 1 to 10 carbon atoms, whereas 1 carbon atom is replaced by oxygen.

6. Compounds according to claim 4, wherein Y is linear hydrocarbon chain containing 1 to 3 carbon atoms.

7. Compounds according to any one of the claims 4 to 7, wherein substituents R are independently selected from the group consisting of H; ¹²⁷I; C₆ to C₁₀ aryl; C₁ to C₁₄ alkoxy; benzyloxy; NO₂.

8. Compounds according to claim 4, selected from the group comprising: (2-hydroxyethyl)-[2-(3-iodo-4-methoxyphenyl)ethyl]-dimethylammonium 4-methylbenzenesulfonate, (2-hydroxyethyl)-[2-(3.5-diiodo-4-methoxyphenyl)ethyl]-dimethylammonium 4-methylbenzenesulfonate, (2-hydroxyethyl)-[3-(3-iodo-4-methoxyphenyl)propyl]-dimethylammonium 4-methylbenzenesulfonate, (2-hydroxyethyl)-[2-(4-iodophenyl)ethyl]-dimethylammonium 4-methylbenzenesulfonate, 1-(2-hydroxyethyl)-1-[2-(4-iodophenyl)ethyl]pyrrolidin-1-ium 4-methylbenzenesulfonate, (2-hydroxyethyl)-(3-iodobenzyl)-dimethylammonium methanesulfonate, 1-(2-hydroxyethyl)-1-(3-iodobenzyl)pyrrolidin-1-ium methanesulfonate, (2-hydroxyethyl)-(4-iodobenzyl)-dimethylammonium bromide, diethyl-(2-hydroxyethyl)-(4-iodobenzyl)ammonium bromide, 1-(2-hydroxyethyl)-1-(4-iodobenzyl)pyrrolidin-1-ium bromide, 1-(2-hydroxyethyl)-1-(2-iodobenzyl)piperidin-1-ium 4-methylbenzenesulfonate, diethyl-(2-hydroxyethyl)-(2-iodobenzyl)ammonium 4-methylbenzenesulfonate, 1,4-bis(2-hydroxyethyl)-1-(2-iodobenzyl)piperazin-1-ium 4-methylbenzenesulfonate, [3-(4-hexyloxy-3-iodophenyl)propyl]-(2-hydroxyethyl)-dimethylammonium 4-methylbenzensulfonate, [3-(4-decyloxy-3-iodophenyl)propyl]-(2-hydroxyethyl)-dimethylammonium methanesulfonate, (2-hydroxyethyl)-[2-(2-iodophenoxy)ethyl]-dimethylammonium bromide, 1-(2-hydroxyethyl)-1-[2-(2-iodophenoxy)ethyl]pyrrolidin-1-ium bromide, (2-hydroxyethyl)-[2-(4-iodophenoxy)ethyl]-dimethylammonium bromide, 1-(2-hydroxyethyl)-1-[2-(4-iodophenoxy)ethyl]pyrrolidin-1-ium bromide, (2-hydroxyethyl)-[2-(3-iodophenoxy)ethyl]-dimethylammonium bromide, 1-(2-hydroxyethyl)-1-[2-(3-iodophenoxy)ethyl]pyrrolidin-1-ium bromide, (2-hydroxyethyl)-[6-(3-iodophenoxy)hexyl]-dimethylammonium bromide, 1-(2-hydroxyethyl)-1-[6-(3-iodophenoxy)hexyl]pyrrolidin-1-ium bromide, (2-hydroxyethyl)-[4-(2-iodophenoxy)butyl]-dimethylammonium bromide, 1-(2-hydroxyethyl)-1-[4-(2-iodophenoxy)butyl]pyrrolidin-1-ium bromide, (2-hydroxyethyl)-[2-(5-iodothiophen-2-yl)ethyl]-dimethylammonium 4-methylbenzenesulfonate, 1-(2-hydroxyethyl)-1-[2-(5-iodothiophen-2-yl)ethyl]pyrrolidin-1-ium 4-methylbenzenesulfonate, 4-(2-hydroxyethyl)-4-(4-iodobenzyl)morpholin-4-ium bromide, 4-(2-hydroxyethyl)-4-(2-iodobenzyl)morpholin-4-ium bromide, (2-hydroxyethyl)-[6-(4-iodo-3-nitrophenoxy)hexyl]-dimethylammonium bromide.

9. A pharmaceutical preparation comprising at least one compound of general formula I according to any one of claims 1 to 3, optionally in the form of pharmaceutically acceptable salt or solvate, and at least one pharmaceutically acceptable carrier, filler and/or diluent.

10. Compounds according to any one of claims 1 to 3 for use as medicaments and/or diagnostic agents.

11. Iodinated aromatic choline analogs of general formula I according to claim 1 for use in a method for radiotherapy and/or radiodiagnostics of disorders involving pathological function or expression of choline transporters selected from the group consisting of oncologic diseases, such as prostate carcinoma, lung carcinoma, glioma; inflammatory disorders; atherosclerosis.

12. Use of the compounds of general formula I according to claim 1 for *in vitro* radiodiagnostics.

13. Iodinated aromatic choline analogs of general formula Ia according to claim 4, selected from the group consisting of: [3-(4-hexyloxy-3-iodophenyl)propyl]-(2-hydroxyethyl)-dimethylammonium 4-methylbenzensulfonate, [3-(4-decyloxy-3-iodophenyl)propyl]-(2-hydroxyethyl)-dimethylammonium methanesulfonate, (2-hydroxyethyl)-[6-(3-iodophenoxy)hexyl]-dimethylammonium bromide, 1-(2-hydroxyethyl)-1-[6-(3-iodophenoxy)hexyl]pyrrolidin-1-ium bromide, (2-hydroxyethyl)-[2-(5-iodothiophen-2-yl)ethyl]-dimethylammonium 4-methylbenzenesulfonate, (2-hydroxyethyl)-{4-[(3-iodo-[1,1'-biphenyl]-4-yl)oxy]butyl}-dimethylammonium bromide, (2-hydroxyethyl)-[6-(4-iodo-3-nitrophenoxy)hexyl]-dimethylammonium bromide, for use as medicaments.

## Patentansprüche

1. Jodierte aromatische Cholin-Analoga der allgemeinen Formel I 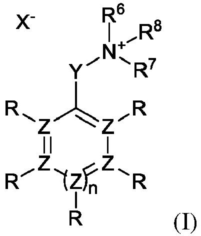 worin
- der Z-Atome enthaltende Ring aus Benzolring und Thiophenring ausgewählt ist;
- R unabhängig ausgewählt sind aus der Gruppe bestehend aus H; ¹²³I; ¹²⁴I; ¹²⁵I; ¹²⁷I; ¹³¹I; C₁-bis C₁₄-Alkyl; C₂- bis C₁₄-Alkenyl; C₂- bis C₁₄-Alkinyl; C₆- bis C₁₀-Aryl; C₃- bis C₆-Heteroaryl, umfassend mindestens ein Heteroatom ausgewählt aus O, S, N; C₃- bis C₆-Heterocyclyl, umfassend ein bis zwei Heteroatome ausgewählt aus O, S, N; C₁- bis C₁₄-Alkoxy; C₆- bis C₁₀-Aryloxy; C₃- bis C₆-Heteroaryloxy, umfassend mindestens ein Heteroatom ausgewählt aus O, S, N; C₃- bis C₆-Heterocyclyloxy, umfassend ein bis zwei Heteroatome ausgewählt aus O, S, N; Benzyloxy; C₁- bis C₁₄-Alkylthio; Halogen; OH; SH; NH₂; C₁- bis C₁₄-Alkylamino; C₆- bis C₁₀-Arylamino; C₁- bis C₁₄-Acylamino; Di(C₁- bis C₁₄-alkyl)amino, wobei die Alkyle gleich oder verschieden sind; Di(C₆- bis C₁₀-aryl)amino, wobei die Aryle gleich oder verschieden sind; Di(C₁- bis C₁₄-acyl)amino, wobei die Acyle gleich oder verschieden sind; CN; Nitro und COORₙ, wobei Rₙ H oder C₁- bis C₁₄-Alkyl oder Aryl ist;
- worin mindestens einer der Substituenten R ¹²³I; ¹²⁴I; ¹²⁵I oder ¹³¹I ist,
- R⁶ 2-Hydroxyethyl, 3-Hydroxypropyl oder 4-Hydroxybutyl ist;
- R⁷ und R⁸ unabhängig ausgewählt sind aus der Gruppe bestehend aus C₁- bis C₆-Alkyl; C₂-bis C₆-Alkenyl; C₂- bis C₆-Alkinyl; C₃- bis C₈-Cycloalkyl; C₃- bis C₈-Cycloalkenyl; C₆- bis C₁₀-Aryl; C₃- bis C₆-Heteroaryl, umfassend mindestens ein Heteroatom ausgewählt aus O, S, N; C₃- bis C₆-Heterocyclyl, umfassend ein oder zwei Heteroatome ausgewählt aus O, S, N; worin R⁷ und R⁸ gegebenenfalls durch eine OH-Gruppe oder eine C₁-C₄-Hydroxyalkylgruppe substituiert sein können; oder
- R⁷ und R⁸ zusammen mit dem Stickstoffatom einen vier- bis siebengliedrigen Ring bilden, der gegebenenfalls mindestens ein weiteres Heteroatom ausgewählt aus O, S, N enthält, und gegebenenfalls durch OH-Gruppe oder C₁-C₄ Hydroxyalkylgruppe substituiert ist;
- worin mindestens einer, vorzugsweise genau einer der Substituenten R⁶, R⁷ und R⁸ mit einer OH-Gruppe oder einer C₁-C₄-Hydroxyalkylgruppe substituiert ist;
- Y ein Linker ist, der von einer linearen oder verzweigten Kohlenwasserstoffkette mit 1 bis 10 Kohlenstoffatomen gebildet wird, worin gegebenenfalls 1 bis 2 Kohlenstoffatome durch Sauerstoff- und/oder Schwefelatome ersetzt sein können; und
- X⁻ ein pharmazeutisch verträgliches Anion ist;
mit der Maßgabe, dass:
- wenn genau einer der Substituenten R ¹²³I; ¹²⁴I; ¹²⁵I oder ¹³¹I ist und steht in *ortho-Stellung,* und alle anderen Substituenten R sind Wasserstoffatome, Y ist -CH₂-, R⁶ ist 2-Hydroxyethyl, dann ist NR⁷R⁸ keine Dimethylamino- oder Pyrrolidinogruppe,
- wenn genau einer der Substituenten R ¹²³I; ¹²⁴I; ¹²⁵I oder ¹³¹I ist und in *ortho-Stellung* steht und alle anderen Substituenten R Wasserstoffatome sind, Y -CH2- ist, R⁶ 2-Hydroxyethyl ist, R⁷ Ethyl oder 2-Hydroxyethyl ist, dann R⁸ kein Methyl ist.

2. Verbindungen nach Anspruch 1, wobei Y eine lineare oder verzweigte Kohlenwasserstoffkette mit 1 bis 10 Kohlenstoffatomen ist, wobei 1 Kohlenstoffatom durch Sauerstoffatom ersetzt ist; oder wobei Y eine lineare Kohlenwasserstoffkette mit 1 bis 3 Kohlenstoffatomen ist.

3. Verbindungen nach einem der vorhergehenden Ansprüche, wobei die Substituenten R unabhängig ausgewählt sind aus der Gruppe bestehend aus H; ¹²³I; ¹²⁴I; ¹²⁵I, ¹²⁷I; ¹³¹I; C₆- bis C₁₀-Aryl; C₁- bis C₁₄-Alkoxy; Benzyloxy; NO₂.

4. Jodierte aromatische Cholin-Analoga der allgemeinen Formel Ia 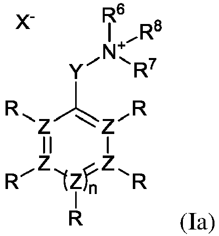 worin
- der Z-Atome enthaltende Ring aus Benzolring und Thiophenring ausgewählt ist;
- R unabhängig ausgewählt sind aus der Gruppe bestehend aus H; ¹²⁷I; C₁- bis C₁₄-Alkyl; C₂-bis C₁₄-Alkenyl; C₂- bis C₁₄-Alkinyl; C₆- bis C₁₀-Aryl; C₃- bis C₆-Heteroaryl, umfassend mindestens ein Heteroatom ausgewählt aus O, S, N; C₃- bis C₆-Heterocyclyl, umfassend ein bis zwei Heteroatome ausgewählt aus O, S, N; C₁- bis C₁₄-Alkoxy; C₆- bis C₁₀-Aryloxy; C₃- bis C₆-Heteroaryloxy, umfassend mindestens ein Heteroatom ausgewählt aus O, S, N; C₃- bis C₆-Heterocyclyloxy, umfassend ein bis zwei Heteroatome ausgewählt aus O, S, N; Benzyloxy; C₁-bis C₁₄-Alkylthio; Halogen; OH; SH; NH₂; C₁- bis C₁₄-Alkylamino; C₆- bis C₁₀-Arylamino; C₁-bis C₁₄-Acylamino; Di(C₁- bis C₁₄-alkyl)amino, wobei die Alkyle gleich oder verschieden sind; Di(C₆- bis C₁₀-aryl)amino, wobei die Aryle gleich oder verschieden sind; Di(C₁- bis C₁₄-acyl)amino, wobei die Acyle gleich oder verschieden sind; CN; Nitro und COORₙ, wobei Rₙ H oder C₁- bis C₁₄-Alkyl oder Aryl ist;
- worin mindestens einer der Substituenten R ¹²⁷I ist,
- R⁶ 2-Hydroxyethyl, 3-Hydroxypropyl oder 4-Hydroxybutyl ist;
- R⁷ und R⁸ unabhängig ausgewählt sind aus der Gruppe bestehend aus C₁- bis C₆-Alkyl; C₂-bis C₆-Alkenyl; C₂- bis C₆-Alkinyl; C₃- bis C₈-Cycloalkyl; C₃- bis C₈-Cycloalkenyl; C₆- bis C₁₀-Aryl; C₃- bis C₆-Heteroaryl, umfassend mindestens ein Heteroatom ausgewählt aus O, S, N; C₃- bis C₆-Heterocyclyl, umfassend ein oder zwei Heteroatome ausgewählt aus O, S, N; worin R⁷ und R⁸ gegebenenfalls durch eine OH-Gruppe oder eine C₁-C₄-Hydroxyalkylgruppe substituiert sein können; oder
- R⁷ und R⁸ zusammen mit dem Stickstoffatom einen vier- bis siebengliedrigen Ring bilden, der gegebenenfalls mindestens ein weiteres Heteroatom ausgewählt aus O, S, N enthält, und gegebenenfalls durch OH-Gruppe oder C1-C4 Hydroxyalkylgruppe substituiert ist; oder
- worin mindestens einer, vorzugsweise genau einer der Substituenten R⁶, R⁷ und R⁸ mit einer OH-Gruppe oder einer C₁-C₄-Hydroxyalkylgruppe substituiert ist;
- Y ein Linker ist, der von einer linearen oder verzweigten Kohlenwasserstoffkette mit 1 bis 10 Kohlenstoffatomen gebildet wird, worin gegebenenfalls 1 Kohlenstoffatome durch Sauerstoff- oder Schwefelatome ersetzt sein können; und
- X⁻ ein pharmazeutisch verträgliches Anion ist;
mit der Maßgabe, dass:
- wenn genau einer der Substituenten R ¹²⁷I ist und steht in *ortho-Stellung,* und alle anderen Substituenten R sind Wasserstoffatome, Y ist -CH₂-, R⁶ ist 2-Hydroxyethyl, dann ist NR⁷R⁸ keine Dimethylamino- oder Pyrrolidinogruppe,
- wenn genau einer der Substituenten R ¹²⁷I ist und in *ortho-Stellung* steht und alle anderen Substituenten R Wasserstoffatome sind, Y -CH2- ist, R⁶ 2-Hydroxyethyl ist, R⁷ Ethyl oder 2-Hydroxyethyl ist, dann R⁸ kein Methyl ist.

5. Verbindungen nach Anspruch 4, wobei Y eine lineare oder verzweigte Kohlenwasserstoffkette mit 1 bis 10 Kohlenstoffatomen ist, wobei 1 Kohlenstoffatom durch Sauerstoffatom ersetzt ist.

6. Verbindungen nach Anspruch 4, worin Y eine lineare Kohlenwasserstoffkette mit 1 bis 3 Kohlenstoffatomen ist.

7. Verbindungen nach einem der Ansprüche 4 bis 7, wobei die Substituenten R unabhängig ausgewählt sind aus der Gruppe bestehend aus H; ¹²⁷I; C₆- bis C₁₀-Aryl; C₁- bis C₁₄-Alkoxy; Benzyloxy; NO₂.

8. Verbindungen nach Anspruch 4, ausgewählt aus der Gruppe umfassend: (2-Hydroxyethyl)-[2-(3-jod-4-methoxyphenyl)ethyl]-dimethylammonium-4-methylbenzolsulfonat, (2-Hydroxyethyl)-[2-(3,5-dijod-4-methoxyphenyl)ethyl]-dimethylammonium-4-methylbenzolsulfonat, (2-Hydroxyethyl)-[3-(3-jod-4-methoxyphenyl)propyl]-dimethylammonium-4-methylbenzolsulfonat, (2-Hydroxyethyl)-[2-(4-jodphenyl)ethyl]-dimethylammonium-4-methylbenzolsulfonat, 1-(2-Hydroxyethyl)-1-[2-(4-jodphenyl)ethyl]pyrrolidin-l-ium-4-methylbenzolsulfonat, (2-Hydroxyethyl)-(3-jodbenzyl)-dimethylammoniummethansulfonat, 1-(2-Hydroxyethyl)-1-(3-jodbenzyl)pyrrolidin-1-iummethansulfonat, (2-Hydroxyethyl)-(4-jodbenzyl)-dimethylammoniumbromid, Diethyl-(2-hydroxyethyl)-(4-jodbenzyl)ammoniumbromid, 1-(2-Hydroxyethyl)-1-(4-jodbenzyl)pyrrolidin-l-iumbromid, 1-(2-Hydroxyethyl)-1-(2-jodbenzyl)piperidin-1-ium-4-methylbenzolsulfonat, Diethyl-(2-hydroxyethyl)-(2-jodbenzyl)ammonium-4-methylbenzolsulfonat, 1,4-Bis(2-hydroxyethyl)-1-(2-jodbenzyl)piperazin-1-ium-4-methylbenzol sulfonat, [3-(4-Hexyloxy-3-jodphenyl)propyl]-(2-hydroxyethyl)-dimethylammonium-4-methylbenzolsulfonat, [3-(4-Decyloxy-3-jodphenyl)propyl]-(2-hydroxyethyl)-dimethylammoniummethansulfonat, (2-Hydroxyethyl)-[2-(2-jodphenoxy)ethyl]-dimethylammoniumbromid, 1-(2-Hydroxyethyl)-1-[2-(2-jodphenoxy)ethyl]pyrrolidin-1-iumbromid, (2-Hydroxyethyl)-[2-(4-jodphenoxy)ethyl]-dimethylammoniumbromid, 1-(2-Hydroxyethyl)-1-[2-(4-jodphenoxy)ethyl]pyrrolidin-1-iumbromid, (2-Hydroxyethyl)-[2-(3-jodphenoxy)ethyl]-dimethylammoniumbromid, 1-(2-Hydroxyethyl)-1-[2-(3-jodphenoxy)ethyl]pyrrolidin-1-iumbromid, (2-Hydroxyethyl)-[6-(3-jodphenoxy)hexyl]-dimethylammoniumbromid, 1-(2-Hydroxyethyl)-1-[6-(3-jodphenoxy)hexyl]pyrrolidin-1-iumbromid, (2-Hydroxyethyl)-[4-(2-jodphenoxy)butyl]-dimethylammoniumbromid, 1-(2-Hydroxyethyl)-1-[4-(2-jodphenoxy)butyl]pyrrolidin-1-iumbromid, (2-Hydroxyethyl)-[2-(5-jodthiophen-2-yl)ethyl]-dimethylammonium-4-methylbenzolsulfonat, 1-(2-Hydroxyethyl)-1-[2-(5-jodthiophen-2-yl)ethyl]pyrrolidin-1-ium-4-methylbenzolsulfonat, 4-(2-Hydroxyethyl)-4-(4-jodbenzyl)morpholin-4-iumbromid, 4-(2-Hydroxyethyl)-4-(2-jodbenzyl)morpholin-4-iumbromid, (2-Hydroxyethyl)-[6-(4-jod-3-nitrophenoxy)hexyl]-dimethylammoniumbromid.

9. Pharmazeutisches Präparat, enthaltend mindestens eine Verbindung der allgemeinen Formel I nach einem der Ansprüche 1 bis 3, gegebenenfalls in Form eines pharmazeutisch verträglichen Salzes oder Solvats, und mindestens einen pharmazeutisch verträglichen Träger, Füllstoff und/oder Verdünnungsmittel.

10. Verbindungen nach einem der Ansprüche 1 bis 3 zur Verwendung als Arzneimittel und/oder Diagnostika.

11. Jodierte aromatische Cholin-Analoga der allgemeinen Formel I nach Anspruch 1 zur Verwendung in einem Verfahren zur Strahlentherapie und/oder Strahlendiagnostik von Erkrankungen mit pathologischer Funktion oder Expression von Cholintransportern ausgewählt aus der Gruppe bestehend aus onkologischen Erkrankungen, wie Prostatakarzinom, Lungen-Karzinom, Gliom; entzündliche Störungen; Arteriosklerose.

12. Verwendung der Verbindungen der allgemeinen Formel I gemäß Anspruch 1 für die *in vitro* Strahlendiagnostik.

13. Jodierte aromatische Cholin-Analoga der allgemeinen Formel Ia nach Anspruch 4, ausgewählt aus der Gruppe bestehend aus: [3-(4-Hexyloxy-3-jodphenyl)propyl] -(2-hydroxyethyl)-dimethylammonium-4-methylbenzolsulfonat, [3-(4-Decyloxy-3-jodphenyl)propyl]-(2-hydroxyethyl)-dimethylammoniummethansulfonat, (2-Hydroxyethyl)-[6-(3-jodphenoxy)hexyl]-dimethylammoniumbromid, 1-(2-Hydroxyethyl)-1-[6-(3-jodphenoxy)hexyllpyrrolidin-1-iumbromid, (2-Hydroxyethyl)-[2-(5-jodthiophen-2-yl)ethyl]-dimethylammonium-4-methylbenzolsulfonat, (2-Hydroxyethyl-{4-[(3 -jod-[1,1'-biphenyl]-4-yl)oxy]butyl}-dimethylammoniumbromid, (2-Hydroxyethyl)-[6-(4-jod-3-nitrophenoxy)hexyl]-dimethylammoniumbromid, zur Verwendung als Medikamente.

## Revendications

1. Analogues aromatiques iodés de la choline de formule générale I 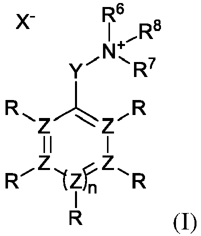 dans laquelle
- le cycle contenant des atomes Z est choisi parmi le cycle benzénique et le cycle thiophène ;
- R sont choisis indépendamment dans le groupe constitué par H ; ¹²³I; ¹²⁴I; ¹²⁵I; ¹²⁷I; ¹³¹I; alkyle en C₁ à C₁₄; alcényle en C₂ à C₁₄; alcynyle en C₂ à C₁₄; aryle en C₆ à C₁₀ ; hétéroaryle en C₃ à C₆ comprenant au moins un hétéroatome choisi parmi O, S, N ; hétérocyclyle en C₃ à C₆ comprenant un à deux hétéroatomes choisis parmi O, S, N ; alcoxy en C₁ à C₁₄; aryloxy en C₆ à C₁₀; hétéroaryloxy en C₃ à C₆ comprenant au moins un hétéroatome choisi parmi O, S, N ; hétérocyclyloxy en C₃ à C₆ comprenant un à deux hétéroatomes choisis parmi O, S, N; benzyloxy; alkylthio en C₁ à C₁₄; halogène; OH; SH; NH₂; alkylamino en C₁ à C₁₄; arylamino en C₆ à C₁₀; acylamino en C₁ à C₁₄; di(C₁ à C₁₄ alkyl)amino, dans lequel les alkyles sont identiques ou différents; di(C₆ à C₁₀ aryl)amino, dans lequel les aryles sont identiques ou différents; di(C₁ à C₁₄ acyl)amino, où les acyles sont identiques ou différents; CN; nitro et COORn, où Rₙ est H ou alkyle ou aryle en C₁ à C₁₄;
- alors qu'au moins un des substituants R est ¹²³I ; ¹²⁴I; ¹²⁵I ou ¹³¹I,
- R⁶ est 2-hydroxyéthyle, 3-hydroxypropyle ou 4 hydroxybutyle ;
- R⁷ et R⁸ sont choisis indépendamment dans le groupe constitué par les groupes alkyle en C₁ à C₆ ; alcényle en C₂ à C₆; alcynyle en C₂ à C₆; cycloalkyle en C₃ à C₈; cycloalcényle en C₃ à C₈; aryle en C₆ à C₁₀ ; hétéroaryle en C₃ à C₆ comprenant au moins un hétéroatome choisi parmi O, S, N ; hétérocyclyle en C₃ à C₆ comprenant un ou deux hétéroatomes choisis parmi O, S, N ; tandis que R⁷ et R⁸ peuvent éventuellement être substitués par un groupe OH ou un groupe hydroxyalkyle en C₁-C₄ ; ou alors
- R⁷ et R⁸ forment avec l'atome d'azote un cycle de quatre à sept chaînons, comprenant éventuellement au moins un autre hétéroatome choisi parmi O, S, N, un groupe éventuellement substitué par un groupe OH ou un groupe hydroxyalkyle en C₁-C₄ ;
- alors qu'au moins un, de préférence exactement un, des substituants R⁶, R⁷ et R⁸ est substitué par un groupe OH ou par un groupe hydroxyalkyle en C₁-C₄ ;
- Y est un lieur formé d'une chaîne hydrocarbonée linéaire ou ramifiée ayant de 1 à 10 atomes de carbone, alors qu'éventuellement 1 à 2 atomes de carbone peuvent être remplacés par des atomes d'oxygène et/ou de soufre ; et
- X⁻ est un anion pharmaceutiquement acceptable ;
à condition que :
- quand exactement l'un des substituants R est ¹²³I ; ¹²⁴I; ¹²⁵I ou ¹³¹I, et est en position ortho, et tous les autres substituants R sont des atomes d'hydrogène, Y est -CH₂-, R⁶ est 2-hydroxyéthyle, alors NR⁷R⁸ n'est pas un groupe diméthylamino ou pyrrolidino,
- quand exactement l'un des substituants R est ¹²³I ; ¹²⁴I; ¹²⁵I ou ¹³¹I et est en position ortho, et tous les autres substituants R sont des atomes d'hydrogène, Y est -CH₂-, R⁶ est 2-hydroxyéthyle, R⁷ est éthyle ou 2-hydroxyéthyle, alors R⁸ n'est pas méthyle.

2. Composés selon la revendication 1, dans lesquels Y est une chaîne hydrocarbonée linéaire ou ramifiée ayant de 1 à 10 atomes de carbone, tandis que 1 atome de carbone est remplacé par l'oxygène ; ou dans laquelle Y est une chaîne hydrocarbonée linéaire contenant 1 à 3 atomes de carbone.

3. Composés selon l'une quelconque des revendications précédentes, dans lesquels les substituants R sont choisis indépendamment dans le groupe constitué par H; ¹²³I; ¹²⁴I; ¹²⁵I; ¹²⁷I, ¹³¹I; aryle en C₆ à C₁₀ ; alcoxy en C₁ à C₁₄; benzyloxy; NO₂.

4. Analogues aromatiques iodés de la choline de formule générale la 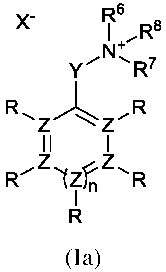 où
- le cycle contenant des atomes Z est choisi parmi le cycle benzénique et le cycle thiophène ;
- R sont choisis indépendamment dans le groupe constitué par H ; ¹²⁷I; alkyle en C₁ à C₁₄; alcényle en C₂ à C₁₄; alcynyle en C₂ à C₁₄; aryle en C₆ à C₁₀ ; hétéroaryle en C₃ à C₆ comprenant au moins un hétéroatome choisi parmi O, S, N ; hétérocyclyle en C₃ à C₆ comprenant un à deux hétéroatomes choisis parmi O, S, N ; alcoxy en C₁ à C₁₄; aryloxy en C₆ à C₁₀; hétéroaryloxy en C₃ à C₆ comprenant au moins un hétéroatome choisi parmi O, S, N ; hétérocyclyloxy en C₃ à C₆ comprenant un à deux hétéroatomes choisis parmi O, S, N; benzyloxy; alkylthio en C₁ à C₁₄; halogène; OH; SH ; NH₂; alkylamino en C₁ à C₁₄; arylamino en C₆ à C₁₀; acylamino en C₁ à C₁₄; di(C₁ à C₁₄ alkyl)amino, dans lequel les alkyles sont identiques ou différents; di(C₆ à C₁₀ aryl)amino, dans lequel les aryles sont identiques ou différents; di(C₁ à C₁₄ acyl)amino, où les acyles sont identiques ou différents; CN; nitro et COORₙ, où Rₙ est H ou alkyle ou aryle en C₁ à C₁₄;
- alors qu'au moins un des substituants R est ¹²⁷I ;
- R⁶ est 2-hydroxyéthyle, 3-hydroxypropyle ou 4 hydroxybutyle ;
- R⁷et R⁸ sont choisis indépendamment dans le groupe constitué par les groupes alkyle en C₁ à C₆ ; alcényle en C₂ à C₆; alcynyle en C₂ à C₆; cycloalkyle en C₃ à C₈; cycloalcényle en C₃ à C₈; aryle en C₆ à C₁₀ ; hétéroaryle en C₃ à C₆ comprenant au moins un hétéroatome choisi parmi O, S, N ; hétérocyclyle en C₃ à C₆ comprenant un ou deux hétéroatomes choisis parmi O, S, N ; tandis que R⁷ et R⁸ peuvent éventuellement être substitués par un groupe OH ou un groupe hydroxyalkyle en C₁-C₄ ; ou alors
- R⁷ et R⁸ forment avec l'atome d'azote un cycle de quatre à sept chaînons, comprenant éventuellement au moins un autre hétéroatome choisi parmi O, S, N, et un groupe éventuellement substitué par un groupe OH ou un groupe hydroxyalkyle en C₁-C₄ ; ou alors
- alors qu'au moins un, de préférence exactement un, des substituants R⁶, R⁷ et R⁸ est substitué par un groupe OH ou par un groupe hydroxyalkyle en C₁-C₄ ;
- Y est un lieur formé d'une chaîne hydrocarbonée linéaire ou ramifiée ayant de 1 à 10 atomes de carbone, alors qu'éventuellement 1 atome de carbone peut être remplacé par un atome d'oxygène ou de soufre ; et
- X⁻ est un anion pharmaceutiquement acceptable ;
à condition que :
- quand exactement l'un des substituants R est ¹²⁷I, et est en position ortho, et que tous les autres substituants R sont des atomes d'hydrogène, Y est -CH₂-, R⁶ est 2-hydroxyéthyle, alors NR⁷R⁸ n'est pas un groupe diméthylamino ou pyrrolidino,
- quand exactement l'un des substituants R est ¹²⁷I et est en position ortho, et que tous les autres substituants R sont des atomes d'hydrogène, Y est -CH₂-, R⁶ est 2-hydroxyéthyle, R⁷ est éthyle ou 2-hydroxyéthyle, alors R⁸ n'est pas méthyle.

5. Composés selon la revendication 4, dans lesquels Y est une chaîne hydrocarbonée linéaire ou ramifiée ayant de 1 à 10 atomes de carbone, tandis que 1 atome de carbone est remplacé par l'oxygène.

6. Composés selon la revendication 4, dans lesquels Y est une chaîne hydrocarbonée linéaire contenant 1 à 3 atomes de carbone.

7. Composés selon l'une quelconque des revendications 4 à 7, dans lesquels les substituants R sont choisis indépendamment dans le groupe constitué par H ; ¹²⁷I; aryle en C₆ à C₁₀ ; alcoxy en C₁ à C₁₄; benzyloxy; NO₂.

8. Composés selon la revendication 4, choisis dans le groupe comprenant : 4-méthylbenzènesulfonate de (2-hydroxyéthyl)-[2-(3-iodo-4-méthoxyphényl)éthyl]-diméthylammonium, 4-méthylbenzènesulfonate de (2-hydroxyéthyl)-[2-(3,5-diiodo-4-méthoxyphényl)éthyl]-diméthylammonium, 4-méthylbenzènesulfonate de (2-hydroxyéthyl)-[3-(3-iodo-4-méthoxyphényl)propyl]-diméthylammonium, 4-méthylbenzènesulfonate de (2-hydroxyéthyl)-[2-(4-iodophényl)éthyl]-diméthylammonium, 4-méthylbenzènesulfonate de 1-(2-hydroxyéthyl)-1-[2-(4-iodophényl)éthyl]pyrrolidin-1-ium, méthanesulfonate de (2-hydroxyéthyl)-(3-iodobenzyl)-diméthylammonium, méthanesulfonate de 1-(2-hydroxyéthyl)-1-(3-iodobenzyl)pyrrolidin-1-ium, bromure de (2-hydroxyéthyl)-(4-iodobenzyl)-diméthylammonium, bromure de diéthyl-(2-hydroxyéthyl)-(4-iodobenzyl)ammonium, bromure de 1-(2-hydroxyéthyl)-1-(4-iodobenzyl)pyrrolidin-1-ium, 4-méthylbenzènesulfonate de 1-(2-hydroxyéthyl)-1-(2-iodobenzyl)pipéridin-1-ium, 4-méthylbenzènesulfonate de diéthyl-(2-hydroxyéthyl)-(2-iodobenzyl)ammonium, 4-méthylbenzènesulfonate de 1,4-bis(2-hydroxyéthyl)-1-(2-iodobenzyl)pipérazin-1-ium, 4-méthylbenzènesulfonate de [3-(4-hexyloxy-3-iodophényl)propyl]-(2-hydroxyéthyl)-diméthylammonium, méthanesulfonate de [3-(4-décyloxy-3-iodophényl)propyl]-(2-hydroxyéthyl)-diméthylammonium, bromure de (2-hydroxyéthyl)-[2-(2-iodophénoxy)éthyl]-diméthylammonium, bromure de 1-(2-hydroxyéthyl)-1-[2-(2-iodophénoxy)éthyl]pyrrolidin-1-ium, bromure de (2-hydroxyéthyl)-[2-(4-iodophénoxy)éthyl]-diméthylammonium, bromure de 1-(2-hydroxyéthyl)-1-[2-(4-iodophénoxy)éthyl]pyrrolidin- 1-ium, bromure de (2-hydroxyéthyl)-[2-(3-iodophénoxy)éthyl]-diméthylammonium, bromure de 1-(2-hydroxyéthyl)-1-[2-(3-iodophénoxy)éthyl]pyrrolidin-1-ium, bromure de (2-hydroxyéthyl)-[6-(3-iodophénoxy)hexyl]-diméthylammonium, bromure de 1-(2-hydroxyéthyl)-1-[6-(3-iodophénoxy)hexyl]pyrrolidin-1-ium, bromure de (2-hydroxyéthyl)-[4-(2-iodophénoxy)butyl]-diméthylammonium, bromure de 1-(2-hydroxyéthyl)-1-[4-(2-iodophénoxy)butyl]pyrrolidin-1-ium, 4-méthylbenzènesulfonate de (2-hydroxyéthyl)-[2-(5-iodothiophén-2-yl)éthyl]-diméthylammonium, 4-méthylbenzènesulfonate de 1-(2-hydroxyéthyl)-1-[2-(5-iodothiophén-2-yl)éthyl]pyrrolidin-1-ium, bromure de 4-(2-hydroxyéthyl)-4-(4-iodobenzyl)morpholin-4-ium, bromure de 4-(2-hydroxyéthyl)-4-(2-iodobenzyl)morpholin-4-ium, bromure de (2-hydroxyéthyl)-[6-(4-iodo-3-nitrophénoxy)hexyl]-diméthylammonium.

9. Préparation pharmaceutique comprenant au moins un composé de formule générale I selon l'une quelconque des revendications 1 à 3, éventuellement sous forme de sel ou solvate pharmaceutiquement acceptable, et au moins un support, charge et/ou diluant pharmaceutiquement acceptable.

10. Composés selon l'une quelconque des revendications 1 à 3 pour utilisation comme médicaments et/ou agents de diagnostic.

11. Analogues aromatiques iodés de la choline de formule générale I selon la revendication 1 pour utilisation dans une méthode de radiothérapie et/ou de radiodiagnostic de troubles impliquant une fonction pathologique ou l'expression de transporteurs de choline choisis dans le groupe constitué par les maladies oncologiques, telles que le carcinome de la prostate, le carcinome de poumon, gliome; troubles inflammatoires; l'athérosclérose.

12. Utilisation des composés de formule générale I selon la revendication 1 pour le radiodiagnostic *in vitro.*

13. Analogues aromatiques iodés de la choline de formule générale la selon la revendication 4, choisis dans le groupe constitué par : [3-(4-hexyloxy-3-iodophényl)propyl]-(2-hydroxyéthyl)-diméthylammonium 4-méthylbenzènesulfonate, [3-(4-décyloxy-3-iodophényl)propyl]-(2-hydroxyéthyl)-diméthylammonium méthanesulfonate, bromure de (2-hydroxyéthyl)-[6-(3-iodophénoxy)hexyl]-diméthylammonium, bromure de 1-(2-hydroxyéthyl)-1-[6-(3-iodophénoxy)hexyl]pyrrolidin-1-ium, (2-hydroxyéthyl)-[2-(5-iodothiophén-2-yl)éthyl]-diméthylammonium 4-méthylbenzènesulfonate, bromure de (2-hydroxyéthyl)-{4-[(3-iodo-[1,1'-biphényl]-4-yl)oxy]butyl}-diméthylammonium, bromure de (2-hydroxyéthyl)-[6-(4-iodo-3-nitrophénoxy)hexyl]-diméthylammonium, pour utilisation comme médicaments.
